(19) Europäisches Patentamt
European Patent Office
Office européen des brevets

(11) **EP 1 598 343 A1**

(12) # EUROPÄISCHE PATENTANMELDUNG

(43) Veröffentlichungstag:
**23.11.2005 Patentblatt 2005/47**

(21) Anmeldenummer: **04011911.7**

(22) Anmeldetag: **19.05.2004**

(51) Int Cl.[7]: **C07D 239/48**, A61K 31/505, A61P 29/00, A61P 31/12, A61P 35/00, A61P 37/00

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(71) Anmelder: **Boehringer Ingelheim International GmbH**
**55216 Ingelheim am Rhein (DE)**

(72) Erfinder:
- **Engelhardt, Harald**
  **2483 Ebreichsdorf (AT)**
- **Reiser, Ulrich**
  **1130 Wien (AT)**
- **Zahn, Stephan Karl**
  **1130 Wien (AT)**
- **Hauptmann, Rudolf**
  **2483 Ebreichsdorf (AT)**
- **Steegmaier, Martin**
  **1120 Wien (AT)**
- **Guertler, Ulrich**
  **1120 Wien (AT)**
- **Hoffmann, Matthias**
  **88441 Mittelbiberach (DE)**
- **Grauert, Matthias**
  **88400 Biberach (DE)**
- **Stadtmueller, Heinz**
  **2754 Gaweinstal (AT)**

(74) Vertreter: **Hammann, Heinz et al**
**c/o Boehringer Ingelheim GmbH,**
**Postfach 200**
**55216 Ingelheim am Rhein (DE)**

(54) **2-Arylaminopyrimidine als PLK Inhibitoren**

(57) Die vorliegende Erfindung umfasst Verbindungen der allgemeinen Formel (1)

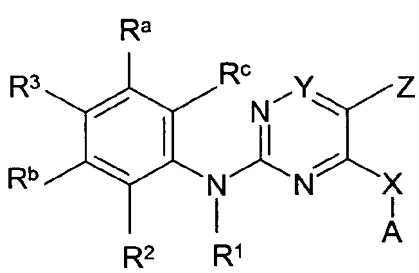

**(1)**

worin
A, X, Y, Z, $R^a$, $R^b$, $R^c$, $R^1$, $R^2$ und $R^3$ wie in Anspruch 1 definiert sind, welche zur Behandlung von Krankheiten, die durch exzessive oder anomale Zellproliferation charakterisiert sind, geeignet sind, sowie deren Verwendung zur Herstellung eines Arzneimittels mit den vorstehend genannten Eigenschaften.

EP 1 598 343 A1

## Beschreibung

Die vorliegende Erfmdung betrifft neue Pyrimidine der allgemeinen Formel (I)

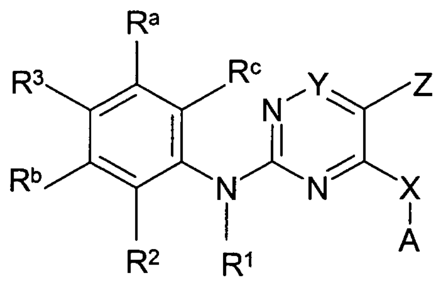

**(1)**

wobei die Reste A, X, Y, Z, $R^a$, $R^b$, $R^c$, $R^1$, $R^2$ und $R^3$ die in den Ansprüchen und der Beschreibung genannten Bedeutungen haben, deren Isomere, Verfahren zur Herstellung dieser Pyrimidine sowie deren Verwendung als Arzneimittel.

## Hintergrund der Erfindung

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).
Studien in Modellorganismen wie *Schizosaccharomyces pombe, Drosophila melanogaster* oder *Xenopus* sowie Untersuchungen in menschlichen Zellen haben gezeigt, dass der Übergang von der G2 Phase zur Mitose durch die CDKl/Cyclin B Kinase reguliert wird (Nurse, 1990). Diese Kinase, die auch als "mitosis promoting factor" (MPF) bezeichnet wird, phosphoryliert und reguliert eine Vielzahl von Proteinen, wie z.B. nukleäre Lamine, Kinesin-ähnliche Motorproteine, Kondensine und Golgi Matrix Proteine, die eine wichtige Rolle beim Abbau der Kernhülle, bei der Zentrosomen Separation, dem Aufbau des mitotischen Spindelapparates, der Chromosomen Kondensation und Abbau des Golgi Apparates spielen (Nigg. E., 2001). Eine murine Zell-Linie mit einer temperatursensitiven CDK-1 Kinasemutante zeigt nach Temperaturerhöhung einen raschen Abbau der CDK-1 Kinase und einen darauf folgenden Arrest in der G2/M Phase (Th'ng et al., 1990). Die Behandlung von humanen Tumorzellen mit Inhibitoren gegen CDKl/Cyclin B, wie z.B. Butyrolacton, führt zu einem Arrest in der G2/M Phase und anschließender Apoptose (Nishio, et al. 1996).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu, J.Y. et al., 2000). Ein spezifischer Zellzyklusarrest in der G2/M Phase kann aber auch z.B. durch Inhibition von spezifischen Phosphatasen wie z.B. Cdc25C (Russell and Nurse, 1986) ausgelöst werden. Hefen mit defektem Cdc25 Gen arretieren in der G2 Phase, während eine Überexpression von Cdc25 zu einem verfrühten Eintritt in die Mitosephase führt (Russell und Nurse, 1987). Desweiteren kann ein Arrest in der G2/M Phase auch durch Inhibition von bestimmten Motorproteinen, den so genannten Kinesinen wie z.B. Eg5 (Mayer et al., 1999), oder durch Mikrotubuli stabilisierende oder destabilisierende Agentien (z.B. Colchicin, Taxol, Etoposid, Vinblastin, Vincristin) ausgelöst werden (Schiff und Horwitz, 1980).

Neben den Cyclin-abhängigen und den Aurora Kinasen spielen des weiteren die so genannten Polo-like Kinasen (PLK), eine kleine Familie von Serin/Threonin-Kinasen, eine wichtige Rolle bei der Regulation des eukaryontischen Zellzykluses. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al., 1998, Qian, et al., 2001). Die Injektion von PLK-1 Antikörpern führt zu einem G2 Arrest in nicht transformierten Zellen, während Tumorzellen in der Mitosephase arretieren (Lane und Nigg, 1996). Überexpression von PLK-1 konnte für verschiedene Tumorarten, wie nicht kleinzelliges Lungenkarzinom, Plattenepithelkarzinom, Brust- und kolorektales Karzinom (10-13) gezeigt werden. Daher stellt diese Klasse von Proteinen ebenfalls einen interessanten Angriffspunkt zur therapeutischen Intervention proliferativer Krankheiten dar (Descombes und Nigg, Embo J, 17; 1).

Pyrimidine sind allgemein als Inhibitoren von Kinasen bekannt. So werden z.B. Pyrimidine als aktive Komponente mit Antikrebswirkung in der Internationalen Patentanmeldung WO 00/53595, in der die Verwendung von 2, 4, 5- substituierten Pyrimidinen mit einem heterozyklischen Rest in 4-Position und einem Anilinorest in 2-Position, der seinerseits eine Seitenkette mit der Länge mindestens eines n-Propylrests aufweist, beschrieben.

Weiterhin wird in der Internationalen Patentanmeldung WO 00/39101 die Verwendung von 2, 4, 5- substituierten Pyrimidinen als Verbindungen mit Antikrebs Wirkung vorgeschlagen, welche in der 2- und 4-Position mit einem aromatischen oder heteroaromatischen Ring verknüpft sind, von denen mindestens einer eine Seitenkette mit der Länge mindestens eines n-Propylrests aufweist.

Die Internationale Patentanmeldung WO 97/19065 schlägt weiterhin die Verwendung von 2, 4, 5- substituierten Pyrimidinen mit einem 3,4-Dialkoxyanilinorest in Position 2 als Kinaseinhibitoren vor.

Die Internationale Patentanmeldung WO 02/04429 beschreibt 2,4,5-substituierte Pyrimidine mit einer Cyanogruppe in Position 5 und deren Zellzyklus inhibierenden Effekt.

In der Internationalen Patentanmeldung WO 03/063794 wird die Verwendung von 2,4-Pyrimidindiaminen als Inhibitoren der IgE und/oder IgG Rezeptor Signalkaskade beschrieben.

Antivirale 2, 4, 5-substituierte Pyrimidine, in denen die Reste $R^c$ und $R^d$ an dem Stickstoff der 4- Position einen heteroaromatischen Fünfring bilden, sind aus der Internationalen Patentanmeldung WO 99/41253 bekannt.

Für 2, 4, 5-substituierte Pyrimidine, die in Position 2 und 4 (Hetero-)Aryle tragen (WO00/27825), sowie für 2, 4, 5-substituierten Pyrimidinen, die in Position 2 oder 4 einen mit einer Nitrilgruppe funktionalisierten (Hetero-)Arylrest tragen (EP 0 945 443 A1), wird eine antivirale Wirkung beschrieben.

Der vorliegenden Erfindung liegt die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können.

## Detaillierte Beschreibung der Erfindung

Es wurde nun überraschenderweise gefunden, dass Verbindungen der allgemeinen Formel (I), worin die Reste A X, Y, $R^a$, $R^b$, $R^c$, $R^1$, $R^2$ und $R^3$ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (**1**)

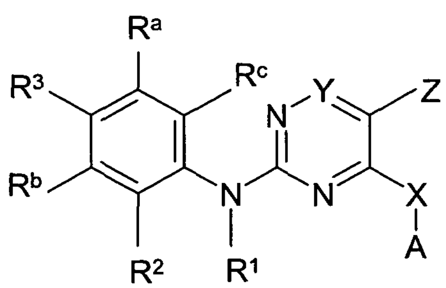

**(1)**

worin

| | |
|---|---|
| **X** | $-NR^{1a}$, O oder S, und |
| **Y** | CH oder N, und |
| **Z** | Wasserstoff, Halogen, $C_{1-3}$-Alkyl, $C_{2-3}$-Alkenyl, $C_{2-3}$-Alkinyl, Halogen-$C_{1-3}$-Alkyl-, -COH, -C(=O)-$C_{1-3}$-Alkyl, -C(=O)-$C_{2-3}$-Alkenyl, -C(=O)-$C_{2-3}$-Alkinyl, -C(=O)$C_{1-3}$-Alkyl-Halogen und Pseudohalogen; und |
| **A** | ausgewählt ist aus den Formeln (i) oder (ii) |

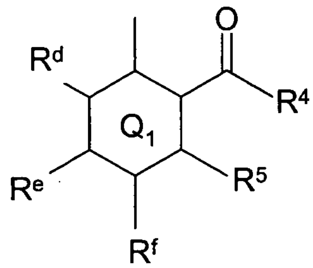

(i)

oder

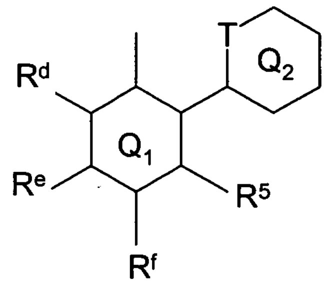

(ii)

und

**$Q_1$** mono- oder bizyklische Arylverbindungen; und

**$Q_2$** mono- oder bizyklische Heteroarylverbindungen; und

**T** N, O oder S, und

**$R^1$ und $R^{1a}$** Wasserstoff oder Methyl, und

**$R^2$** ein Rest ausgewählt aus der Gruppe bestehend aus -Cl, -Br, -I, $-OR^6$, $-C(=O)R^6$, $-C(=O)NR^6R^7$, $-NR^6R^7$, $-NR^6C(=O)R^7$, $-NR^6SO_2R^7$, $-N=CR^6R^7$, $-SR^6$, $-SOR^6$, $-SO_2R^6$, $-SO_2NR^6R^7$ und Pseudohalogen, oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl , Heterocyclyl und Heteroaryl, wobei der/die Substituenten gleich oder verschieden sein können und ausgewählt werden aus der Gruppe bestehend aus Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^a$, $R^b$, $R^c$, $R^d$, $R^e$ und $R^f$** jeweils unabhängig voneinander ein Rest ausgewählt aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen;
oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^3$** ausgewählt aus den Formeln (iii) - (ix),

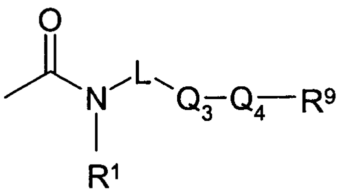
(iii)

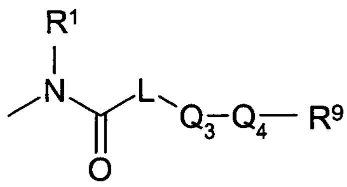
(iv)

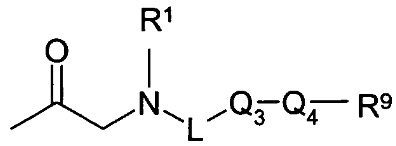
(v)

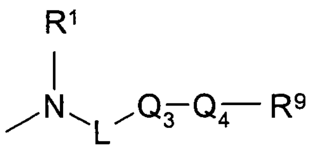
(vi)

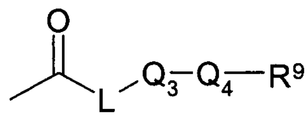
(vii)

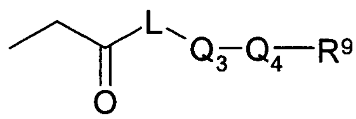
(viii)

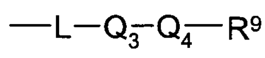
(ix)

und

**$R^4$** ein Rest ausgewählt aus der Gruppe bestehend aus Wasserstoff, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$ $-OSO_2NR^7R^8$ und Pseudohalogen, oder ein Rest ausgewählt aus der Gruppe bestehend aus gegebenenfalls einfach oder mehrfach substituiertem $C_{1-8}$-Alkyl, $C_{2-10}$-Alkenyl, $C_{2-10}$-Alkinyl, $C_{3-8}$-Cycloalkyl, Aryl, Heterocyclyl und Heteroaryl, wobei der/die Substituent(en) gleich oder verschieden sein können und ausgewählt werden aus der Gruppe bestehend aus $C_{1-8}$-Alkyl, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^5$** Wasserstoff, Halogen, $-CF_3$, $C_{1-3}$-Alkyl oder $-OR^6$; und

**$R^6$, $R^7$ und $R^8$** jeweils unabhängig voneinander Wasserstoff oder ein Rest ausgewählt aus der Gruppe bestehend aus gegebenenfalls einfach oder mehrfach substituiertem $C_{1-5}$-Alkyl, $C_{2-5}$-Alkenyl, $C_{2-5}$-Alkinyl, $C_{3-10}$-Cycloalkyl, Aryl, Heterocyclyl und Heteroaryl, wobei der/die Substituenten gleich oder verschieden sein können und ausgewählt werden aus der Gruppe bestehend aus $C_{3-10}$-Cycloalkyl, Aryl, Heterocyclyl, Heteroaryl, Halogen, $-NO_2$, $-OR^{10}$, $-C(=O)R^{10}$, $-C(=O)$

$OR^{10}$, $-C(=O)NR^{10}R^{11}$, $-NR^{10}R^{11}$, $-NR^{10}C(=O)R^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**L** eine Bindung oder ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$Q_3$ und $Q_4$** unabhängig voneinander eine Bindung oder ein Rest ausgewählt aus der Gruppe bestehend aus gegebenenfalls einfach oder mehrfach substitutierten monooder bizyklische Heterocyclyl wobei der/die Substituent(en) gleich oder verschieden sein können und ausgewählt werden aus der Gruppe bestehend aus Methyl, Ethyl, Halogen, $-NH_2$, -OH und Pseudohalogen; und

**$R^9$** ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen-, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$R^{10}$, $R^{11}$ und $R^{12}$** jeweils unabhängig voneinander Wasserstoff oder ein Rest ausgewählt aus der Gruppe bestehend aus gegebenenfalls einfach oder mehrfach substituiertem $C_{1-8}$-Alkyl, $C_{2-8}$-Alkenyl, $C_{2-8}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NH_2$, -OH und Pseudohalogen;

gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, bedeuten.

Ein Aspekt der Erfindung sind Verbindungen der allgemeinen Formel (1), worin

**X** $-NR^{1a}$ oder Sauerstoff, und
**A** ausgewählt ist aus den Formeln (i) oder (ii)

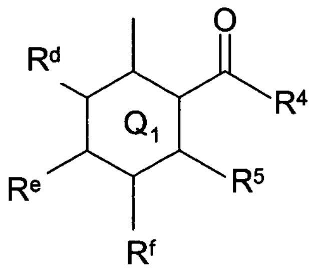

(i)

oder

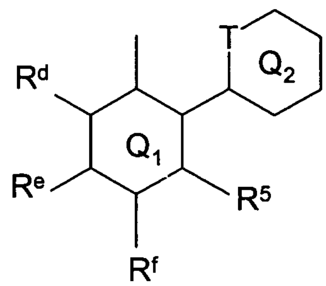

(ii)

und

**$Q_1$** mono- oder bizyklische Arylverbindungen; und

**$Q_2$** monozyklische Heteroarylverbindungen; und

**T** N, O oder S, und

**$R^1$** Wasserstoff; und

**$R^3$** Formel (iii),

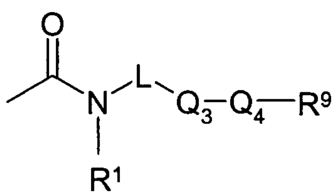

(iii)

bedeuten und die übrigen Reste wie vorstehend erwähnt definiert sind.

Ein weiterer Aspekt der Erfindung sind Verbindungen der allgemeinen Formel (1), worin

**Y** CH; und
**$Q_1$** monozyklische Arylverbindungen

bedeuten und die übrigen Reste wie vorstehend erwähnt definiert sind.

Ein anderen Aspekt der Erfindung sind Verbindungen der allgemeinen Formel (1), worin

**$R^c$** ein Rest ausgewählt aus der Gruppe bestehend aus Wasserstoff, -F, -Cl, Methyl und, Ethyl

bedeutet und die übrigen Reste wie vorstehend erwähnt definiert sind.

Ein zusätzlicher Aspekt dieser Erfindung sind Verbindungen der allgemeinen Formel (1), worin

**$R^a$ und $R^b$** jeweils unabhängig voneinander Wasserstoff oder ein gegebenenfalls einfach oder mehrfach substituierter Rest ausgewählt aus der Gruppe bestehend aus $C_{1-2}$-Alkyl, $C_2$-Alkenyl, $C_2$-Alkinyl, $C_{3-6}$-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6$, $-SO_2NR^7R^8$, $-OSO_2NR^7R^8$ und Pseudohalogen;

bedeuten und die übrigen Reste wie vorstehend erwähnt definiert sind.

Ein Aspekt der Erfindung sind auch Verbindungen der allgemeinen Formel (1), worin $\mathbf{R^a}$ und $\mathbf{R^b}$ Wasserstoff bedeuten und die übrigen Reste wie vorstehend erwähnt definiert sind.

Erfindungsgemäß sind auch Verbindungen der allgemeinen Formel (1) umfasst, worin

**Z** Halogen-$C_{1-3}$-Alkyl-, -COH, -C(=O)-$C_{1-3}$-Alkyl, -C(=O)-$C_{2-3}$-Alkenyl, -C(=O)-$C_{2-3}$-Alkinyl, -C(=O)$C_{1-3}$-Alkyl-Halogen und Pseudohalogen;

bedeutet und die übrigen Reste wie vorstehend erwähnt definiert sind.

Ein weiterer Aspekt der Erfmdung sind Verbindungen der allgemeinen Formel (1), wobei

**Z** $C_{1-3}$-Fluoralkyl und
**Y** CH bedeuten

und die übrigen Reste wie vorsehend erwähnt definiert sind.

Ein Aspekt der Erfindung sind Verbindungen der allgemeinen Formel (1), oder deren pharmazeutisch wirksamen Salze, zur Verwendung als Arzneimittel.

Ein weiterer Aspekt der Erfindung sind Verbindungen der allgemeinen Formel (1), oder deren pharmazeutisch wirksamen Salze, zur Verwendung als Arzneimittel mit antiproliferativer Wirkung.

Ein wesentlicher Aspekt der Erfindung sind Verbindungen der allgemeinen Formel (1), oder deren pharmazeutisch wirksamen Salze, zur Verwendung als Arzneimittel mit antiproliferativer Wirkung.

Ferner sind erfindungsgemäß Verbindungen der allgemeinen Formel (1), oder deren pharmazeutisch wirksamen Salze, zur Verwendung als Arzneimittel mit antiproliferativer Wirkung mit einem selektiven kinaseinhibierenden Wirkmechanismus umfasst.

Ein Aspekt der Erfindung ist die Verwendung von Verbindungen der allgemeinen Formel (1), oder deren pharmazeutisch wirksamen Salze, zur Herstellung eines Arzneimittels mit antiproliferativer Wirkung mit einem PLK inhibierendem Wirkmechanismus.

Ein weiterer Aspekt der Erfindung sind pharmazeutische Zubereitungen, enthaltend als Wirkstoff eine oder mehrere Verbindungen der allgemeinen Formel (I), oder deren physiologisch verträgliche Salze gegebenenfalls in Kombination mit üblichen Hilfs-und/oder Trägerstoffen.

Ein zusätzlicher Aspekt der Erfindung ist die Verwendung einer oder mehrerer Verbindungen der allgemeinen Formel (1) zur Herstellung eines Arzneimittels zur Behandlung und/oder Prävention von Krebs, Infektionen, Entzündungs- und Autoimmunerkrankungen.

Ein anderer Aspekt der Erfindung ist eine pharmazeutische Präperation enthaltend eine Verbindungen der allgemeinen Formel (**1**)

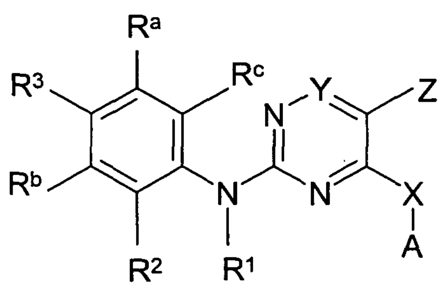

**(1)**

worin

**X** -$NR^{1a}$, O oder S, und

**Y** CH oder N, und

**Z** Wasserstoff, Halogen, $C_{1-3}$-Alkyl, $C_{2-3}$-Alkenyl, $C_{2-3}$-Alkinyl, Halogen-$C_{1-3}$-Alkyl-, -COH, -C(=O)-$C_{1-3}$-Alkyl, -C(=O)-$C_{2-3}$-Alkenyl, -C(=O)-$C_{2-3}$-Alkinyl, -C(=O)$C_{1-3}$-Alkyl-Halogen und Pseudohalogen; und

**A** ausgewählt ist aus den Formeln (i) oder (ii)

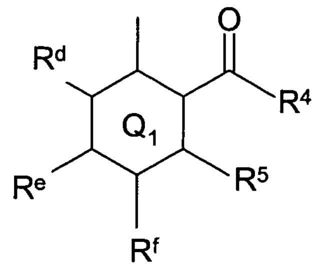

(i)

oder

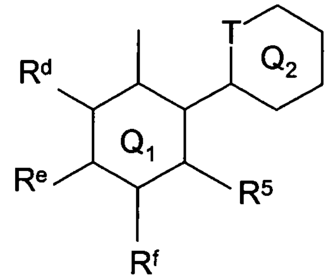

(ii)

und

**$Q_1$** mono- oder bizyklische Arylverbindungen; und

**$Q_2$** mono- oder bizyklische Heteroarylverbindungen; und

**T** N, O oder S, und

**$R^1$** und **$R^{1a}$** Wasserstoff oder Methyl, und

**$R^2$** ein Rest ausgewählt aus der Gruppe bestehend aus -Cl, -Br, -I, $-OR^6$, $-C(=O)R^6$, $-C(=O)NR^6R^7$, $-NR^6R^7$, $-NR^6C(=O)R^7$, $-NR^6SO_2R^7$, $-N=CR^6R^7$, $-SR^6$, $-SOR^6$, $-SO_2R^6$, $-SO_2NR^6R^7$ und Pseudohalogen, oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl , Heterocyclyl und Heteroaryl, wobei der/die Substituenten gleich oder verschieden sein können und ausgewählt werden aus der Gruppe bestehend aus Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^a$, $R^b$, $R^c$, $R^d$, $R^e$** und **$R^f$** jeweils unabhängig voneinander ein Rest ausgewählt aus der Gruppe bestehend aus Wasserstoff, Halogen, $-NO_2$, $-OR^6$, $-C(=O)R^6$, $-C=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen;
oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^3$** ausgewählt aus den Formeln (iii) - (ix),

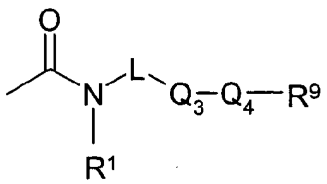

(iii)

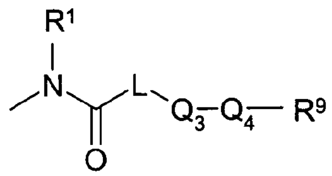

(iv)

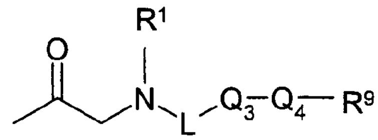

(v)

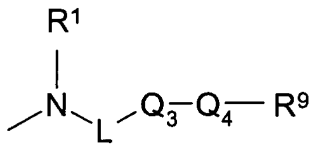

(vi)

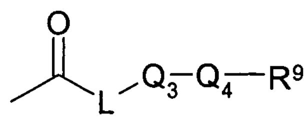

(vii)

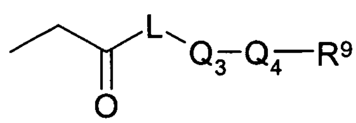

(viii)

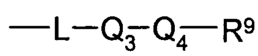

(ix)

und

**$R^4$** ein Rest ausgewählt aus der Gruppe bestehend aus Wasserstoff, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen,
oder ein Rest ausgewählt aus der Gruppe bestehend aus gegebenenfalls einfach oder mehrfach substituiertem $C_{1-8}$-Alkyl, $C_{2-10}$-Alkenyl, $C_{2-10}$-Alkinyl, $C_{3-8}$-Cycloalkyl, Aryl, Heterocyclyl und Heteroaryl, wobei der/die Substituent(en) gleich oder verschieden sein können und ausgewählt werden aus der Gruppe bestehend aus $C_{1-8}$-Alkyl, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^5$** Wasserstoff, Halogen, $-CF_3$, $C_{1-3}$-Alkyl oder $-OR^6$; und

**$R^6$, $R^7$ und $R^8$** jeweils unabhängig voneinander Wasserstoff oder ein Rest ausgewählt aus der Gruppe bestehend aus gegebenenfalls einfach oder mehrfach substituiertem $C_{1-5}$-Alkyl, $C_{2-5}$-Alkenyl, $C_{2-5}$-Alkinyl, $C_{3-10}$-Cycloalkyl, Aryl, Heterocyclyl und Heteroaryl, wobei der/die Substituenten gleich oder verschieden sein können und ausgewählt werden aus der Gruppe bestehend aus $C_{3-10}$-Cycloalkyl, Aryl, Heterocyclyl, Heteroaryl, Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}C(=O)R^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**L** eine Bindung oder ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$Q_3$ und $Q_4$** unabhängig voneinander eine Bindung oder ein Rest ausgewählt aus der Gruppe bestehend aus gegebenenfalls einfach oder mehrfach substitutierten monooder bizyklische Heterocyclyl wobei der/die Substituent(en) gleich oder verschieden sein können und ausgewählt werden aus der Gruppe bestehend aus Methyl, Ethyl, Halogen, $-NH_2$, -OH und Pseudohalogen; und

**$R^9$** ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen-, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$R^{10}$, $R^{11}$ und $R^{12}$** jeweils unabhängig voneinander Wasserstoff oder ein Rest ausgewählt aus der Gruppe bestehend aus gegebenenfalls einfach oder mehrfach substituiertem $C_{1-8}$-Alkyl, $C_{2-8}$-Alkenyl, $C_{2-8}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NH_2$, -OH und Pseudohalogen;

gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und

mindestens eine weitere Wirksubstanz ausgewählt aus der Gruppe bestehend aus zytostatische Wirksubstanzen, zytotoxische Wirksubstanzen, Steroide und Antikörper, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

## DEFINITIONEN

Wie hierin verwendet treffen folgenden Definitionen zu, falls nicht anders beschrieben.

Unter Alkyl-Substitutenten sind jeweils gesättigte, geradkettige oder verzweigte aliphatische Kohlenwasserstoffreste (Alkylrest) zu verstehen.

Die Alkenyl-Substituenten sind jeweils geradkettige oder verzweigte, ungesättigte Alkylreste, die mindestens eine Doppelbindung aufweisen.

Unter Alkinyl-Substituenten sind jeweils geradkettige oder verzweigte, ungesättigte Alkylreste, die mindestens eine Dreifachbindung aufweisen, zu verstehen.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise $-CF_3$, $-CHF_2$; $-CH_2F$, $-CF_2CF_3$,$-CHFCF_3$, $-CH_2CF_3$, $-CF_2CH_3$, $-CHFCH_3$, $-CF_2CF_2CF_3$, $-CF_2CH_2CH_3$, $-CF=CF_2$, $-CCl=CH_2$, $-CBr=CH_2$, $-CJ=CH_2$, $-C\equiv C-CF_3$, $-CHFCH_2CH_3$ und $-CHFCH_2CF_3$.

Halogen bezieht sich auf Fluor-, Chlor-, Brom- und/oder Jodatome.

Unter Pseudohalogen sind folgende Reste zu verstehen: -OCN, -SCN, -CF3 und -CN.

Unter Cycloalkyl ist ein mono- oder bizyklischer Ring zu verstehen, wobei das Ringsystem ein gesättigter Ring aber auch ein ungesättigter, nichtaromatischer Ring sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Norbornyl und Norbornenyl.

Aryl bezieht sich auf monozyklische oder bizyklische Ringe mit 6 - 12 Kohlenstoffatomen wie beispielsweise Phenyl und Naphthyl.

Unter Heteroaryl sind mono- oder bizyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridinyl-N-oxide Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-N-oxide, Pyrimidinyl-N-oxide, Pyridazinyl-N-oxide, Pyrazinyl- N-oxide, Quinolinyl-N-oxide, Indolyl-N-oxide, Indolinyl-N-oxide, Isoquinolyl-N-oxide, Quinazolinyl-N-oxide, Quinoxalinyl-N-oxide, Phthalazinyl-N-oxide, Imidazolyl-N-oxide, Isoxazolyl-N-oxide, Oxazolyl-N-oxide, Thiazolyl-N-oxide, Indolizinyl-N-oxide, Indazolyl-N-oxide, Benzothiazolyl-N-oxide, Benzimidazolyl-N-oxide, Pyrrolyl-N-oxide, Oxadiazolyl-N-oxide, Thiadiazolyl-N-oxide, Triazolyl-N-oxide, Tetrazolyl-N-oxide, Benzothiopyranyl-S-oxide und Benzothiopyranyl-S,S-dioxide.

Heterocyclyl bezieht sich auf 5 -12 Kohlenstoffatome umfassende gesättigte oder ungesättigte, nicht aromatische mono-, bizyklische oder überbrückte bizyklische Ringe, welche anstelle eines oder mehrere Kohlenstoffatome Heteroatome, wie Stickstoff, Sauerstoff oder Schwefel, tragen. Beispiele für solche Heterocylylreste sind Tetrahydrofuranyl, Pyrrolidinyl, Pyrrolinyl, Imidazolidinyl, Imidazolinyl, Pyrazolidinyl, Pyrazolinyl, Piperidyl, Piperazinyl, Indolinyl, Isoindoliny, Morpholinyl, Thiomorpholinyl, Homomorpholinyl, Homopiperidyl, Homopiperazinyl, Thiomorpholinyl-S-oxide, Thiomorpholinyl-S,S-dioxide, Pyrrolidinyl, Pyrrolinyl, Tetrahydropyranyl, Piperidinyl, Tetrahydrofuranyl, Tetrahydrothienyl, Homopiperidinyl, Homothiomorpholinyl-S,S-Dioxide, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-S-oxide, Tetrahydrothienyl-S,S-dioxide, Homothiomorpholinyl-S-oxide, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diazabicyclo[4.2.1]nonan und 2,6-Diaza-bicyclo[3.2.2]nonan.

Die nachfolgenden Beispiele illustrieren die vorliegende Erfindung ohne sie jedoch in ihrem Umfang einzuschränken:

**Herstellung der erfindungsgemäßen Verbindungen:**

Die Herstellung der erfindungsgemäßen Verbindungen kann nach den, im Folgenden beschriebenen Syntheseverfahren A bis C erfolgen, wobei die Substituenten der allgemeinen Formeln **(I** bis **XVI)** die zuvor genannten Bedeutungen haben. Diese Verfahren sind als Erläuterung der Erfindung zu verstehen ohne selbige auf deren Gegenstand zu beschränken.

Chromatographie:

Für die Mitteldruck Chromatographie (MPLC) wird Kieselgel der Firma Millipore (Bezeichnung: Granula Silica Si-60A 35-70μm) oder C-18 RP-Kieselgel der Firma Macherey Nagel (Bezeichnung: Polygoprep 100-50 C18) eingesetzt. Für die Hochdruck Chromatographie (HPLC) werden Säulen der Firma Waters (Bezeichnung: XTerra Prep. MS C18, 5 μM, 30*100 mm oder Symmetrie C18, 5 μm, 19*100) verwendet.

Nuclear Magnet Resonanz (NMR) Spektroskopie:

Die Messung wird in deutoriertem Dimethylsulfoxid-d6 durchgeführt. Werden andere Lösungsmittel verwendet sind diese explizit in den Beispielen oder in den Methoden vermerkt. Die Messwerte werden auf einer Delta-Scala in der Einheit ppm angegeben. Als Standart wird Tetramethylsilan verwendet. Die Messung erfolgt auf einem Avance 400 (400MHz-NMR-Spektrometer) der Firma Bruker Biospin GmbH.

Massenspektroskopie / UV-Spektrometer:

Diese Daten werden mit Hilfe einer HPLC-MS Anlage (high performance liquid chromatography mit Massendetektor) der Firma Agilent erzeugt.
Die Anlage ist so aufgebaut, dass anschließend an die Chromatographie (Säule: Zorbax SB-C8, 3,5 μm, 2,1*50, Fa.

Agilent) ein Diodenarry-Detektor (G1315B von Fa. Agilent) und ein Massendetektor (1100 LS-MSD SL; G1946D; Fa. Agilent) in Reihe geschalten sind.
Die Anlage wird mit einem Fluß von 0,6 ml/min betrieben. Für einen Trennvorgang wird ein Gradient innerhalb von 3,5 min durchlaufen (Gradient Anfang: 95% Wasser und 5% Acetonitril; Gradient Ende: 5% Wasser und 95% Acetonitril; den beiden Lösungsmitteln wird jeweils 0,1 %Ameisensäure beigemischt).

Soweit die Herstellung der Ausgangsverbindungen nicht beschrieben wird, sind diese bekannt, kommerziell erhältlich oder analog zu bekannten Verbindungen oder hier beschriebenen Verfahren herstellbar.

**Verfahren A**

**Stufe 1A**

Die Herstellung der Zwischenverbindung **III** erfolgt durch Substitution einer Abgangsgruppe LG, beispielsweise Halogen, Pseudohalogen, Methoxy, vorzugsweise Chlor, an einem heteroaromatischem System **I** durch ein Nukleophil **II**.

**Schema 1A**

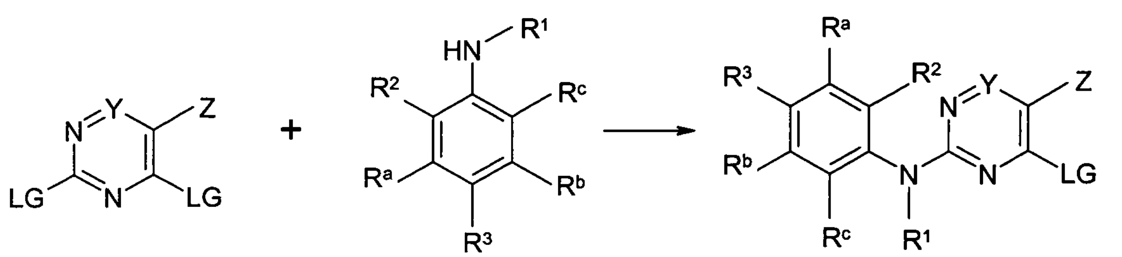

I II III

Es werden 1 Äquivalent der Verbindung **I** und 1 bis 1,5 Äquivalente der Verbindung **II**, in einem Lösungsmittel, beispielsweise 1,4-Dioxan, Tetrahydrofuran, N,N-Dimethylformamid oder N,N-Dimethylacetamid gerührt.
Bei einer Temperatur von 15 bis 25°C werden 2 bis 2,5 Äquivalente einer Base, beispielsweise Kaliumcarbonat, Natriumcarbonat, Cäsiumcarbonat, N-Ethyl-N,N-diisopropylamin oder Triethylamin zugegeben. Das Reaktionsgemisch wird 12 bis 72 h bei einer Temperatur von 15 bis 25°C weitergerührt.
Danach wird das Lösungsmittel abdestilliert und der Rückstand mit Wasser versetzt, welches mit einer Mineralsäure, beispielsweise Salzsäure oder Schwefelsäure auf einen pH-Wert zwischen 1 - 4 eingestellt wird. Dieses Gemisch wird zwei bis dreimal mit einem organischen Lösungsmittel beispielsweise Diethylether, Ethylacetat oder Dichlormethan extrahiert.
Die vereinigten organischen Extrakte werden getrocknet und das Lösungsmittel abdestilliert. Der Rückstand wird chromatographisch gereinigt.

**Stufe 2A**

Die Herstellung der Endverbindung **V** oder **VII** erfolgt durch Substitution einer Abgangsgruppe LG, beispielsweise Halogen, SCN, Methoxy, vorzugsweise Chlor, an einem heteroaromatischem System **III** durch ein Nukleophil **IV** oder **VI.**

## Schema 2A

III +

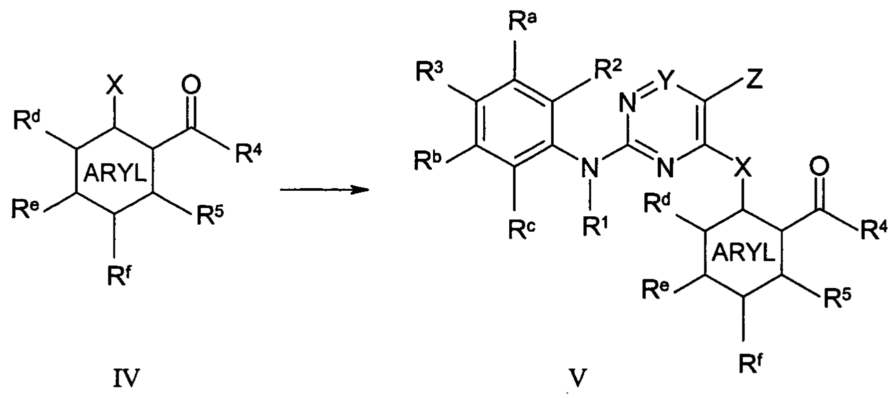

IV V

oder:

III +

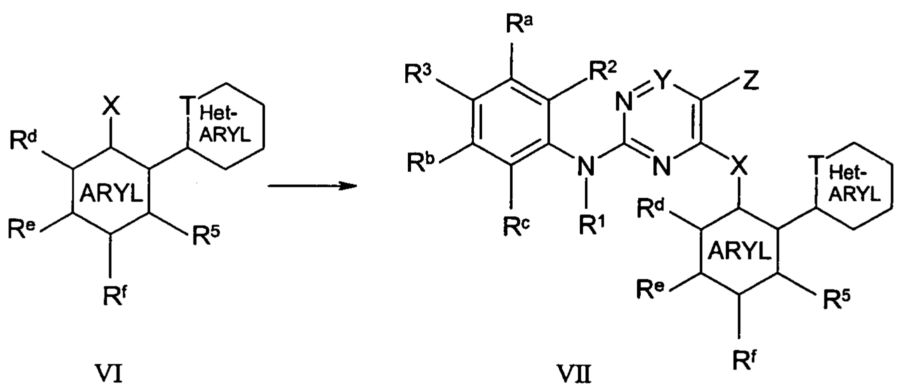

VI VII

Es werden 1 Äquivalent der Verbindung **III** und 1 bis 3 Äquivalente der Verbindung **IV** oder **VI** in einem Lösungsmittel, beispielsweise 1,4-Dioxan, N,N-Dimethylformamid, N,N-Dimethylacetamid oder N-Methyl-2-pyrrolidinon gerührt.
Bei einer Temperatur von 15 bis 40°C werden 1 bis 2 Äquivalente einer Mineralsäure, beispielsweise Schwefelsäure oder Salzsäure zugegeben. Das Reaktionsgemisch wird 12 bis 72 h bei einer Temperatur von 20 bis 100°C weitergerührt.
Danach wird das Lösungsmittel abdestilliert und der Rückstand chromatographisch gereinigt.

### Verfahren B

#### Stufe 1B

Die Herstellung der Zwischenverbindung **IX** erfolgt durch Substitution einer Abgangsgruppe LG, beispielsweise Halogen, SCN, Methoxy, vorzugsweise Chlor, an einem heteroaromatischem System **I** durch ein Nukleophil **VIII.**

## Schema 1B

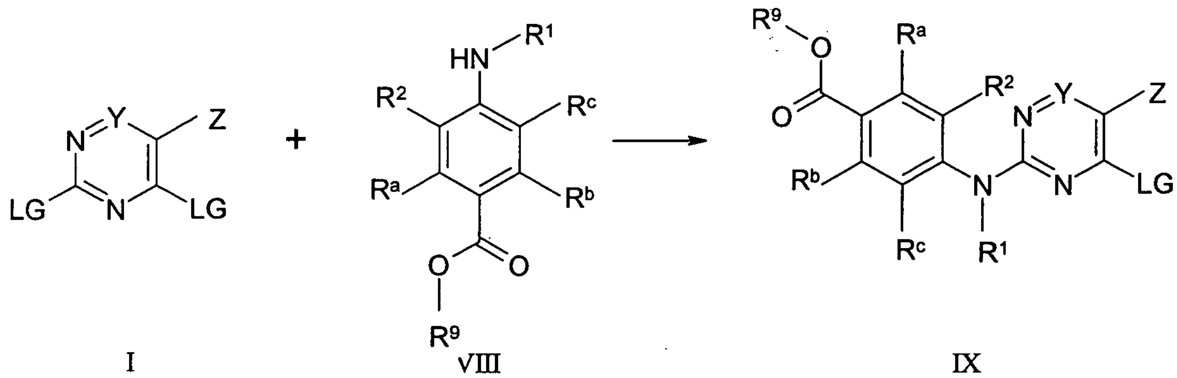

I VIII IX

Es werden 1 Äquivalent der Verbindung I und 1 bis 1,5 Äquivalente der Verbindung **VIII** in einem Lösungsmittel, beispielsweise 1,4-Dioxan, Tetrahydrofuran, N,N-Dimethylformamid oder N,N-Dimethylacetamid gerührt. Bei einer Temperatur von 15 bis 25°C werden 2 bis 2,5 Äquivalente einer Base, beispielsweise Kaliumcarbonat, Natriumcarbonat, Cäsiumcarbonat, Kaliumhydrogenphosphat, N-Ethyl-N,N-diisopropylamin oder Triethylamin zugegeben. Das Reaktionsgemisch wird 2 bis 8 h bei einer Temperatur von 50 bis 120°C weitergerührt.
Das Reaktionsgemisch wird mit Wasser versetzt, welches mit einer anorganischen Base, beispielsweise Natriumhydrogencarbonat oder Kaliumcarbonat auf einen pH-Wert von 8 bis 9 eingestellt wird. Dieses Gemisch wird zwei bis dreimal mit einem organischen Lösungsmittel, beispielsweise Diethylether oder Ethylacetat extrahiert. Die vereinigten organischen Extrakte werden getrocknet und das Lösungsmittel abdestilliert. Der Rückstand wird durch mehrmalige Kristallisation gereinigt.

### Stufe 2B

Die Herstellung der Zwischenverbindung **X** oder **XI** erfolgt durch Substitution einer Abgangsgruppe LG, beispielsweise Halogen, SCN, Methoxy, vorzugsweise Chlor, an einem heteroaromatischem System **IX** durch ein Nukleophil **IV** oder **VI.**

## Schema 2B:

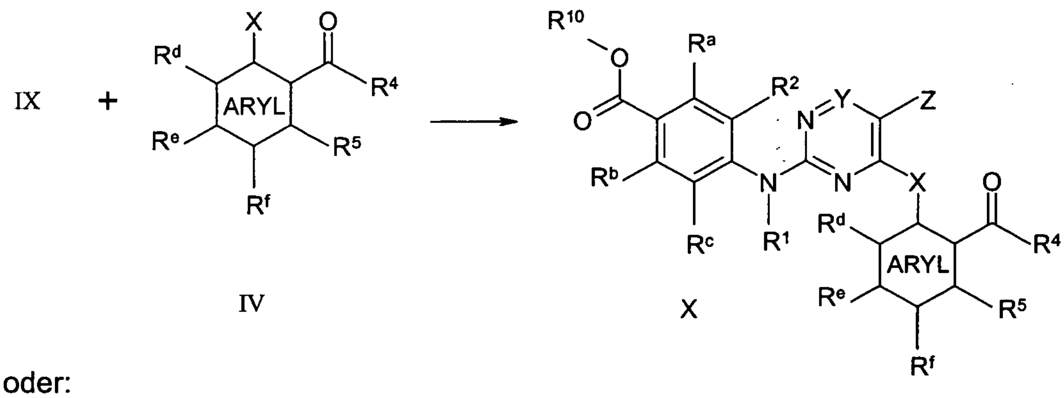

IX

IV

X

oder:

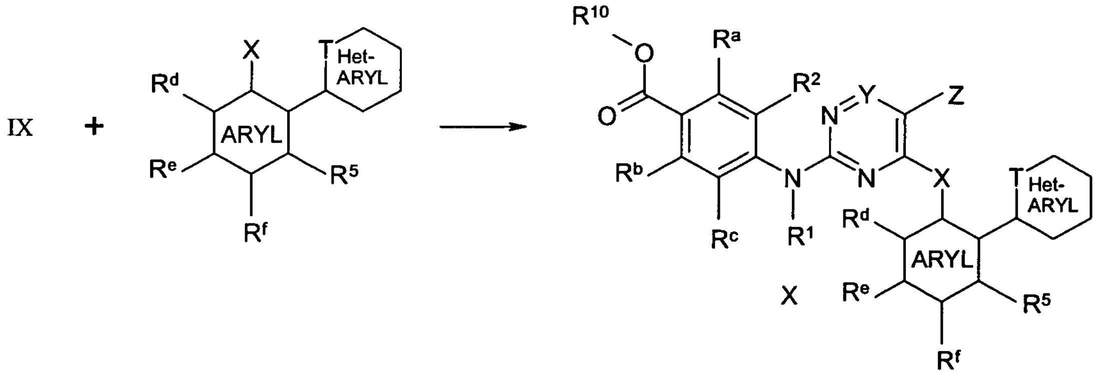

IX

X

VI

XI

Es werden 1 Äquivalent der Verbindung **IX** und 1 bis 1,5 Äquivalente der Verbindung **IV** oder **VI** in einem Lösungsmittel, beispielsweise 1,4-Dioxan, N,N-Dimethylformamid, N,N-Dimethylacetamid oder N-Methyl-2-pyrrolidinon gerührt. Bei einer Temperatur von 15 bis 40°C werden 0,2 bis 1 Äquivalent einer Säure, beispielsweise Schwefelsäure oder Salzsäure zugegeben. Das Reaktionsgemisch wird 12 bis 72 h bei einer Temperatur von 20 bis 100°C weitergerührt.

Das Reaktionsgemisch wird in Wasser eingerührt und der entstehende Niederschlag abfiltriert und getrocknet. Der Niederschlag kann durch Chromatographie oder Kristallisation gereinigt oder als Rohprodukt in der nächsten Stufe eingesetzt werden.

### Stufe 3B

Alle Verbindungen **X** oder **XI** mit einem Rest $R^{10}$ ungleich Wasserstoff, müssen vor der eigentlichen Stufe 3B durch literaturbekannte Verfahren in Verbindungen, bei denen der Rest $R^{10}$ Wasserstoff repräsentiert, überführt werden.

Verbindungen **X** oder **XI** deren Rest $R^{10}$ Wasserstoff darstellt können direkt zur Herstellung der Endverbindungen **XIII** oder **XIV** eingesetzt werden, wobei eine Verbindung **XII** mit einer Verbindung **X** oder **XI** umgesetzt wird.

**Schema 3B**

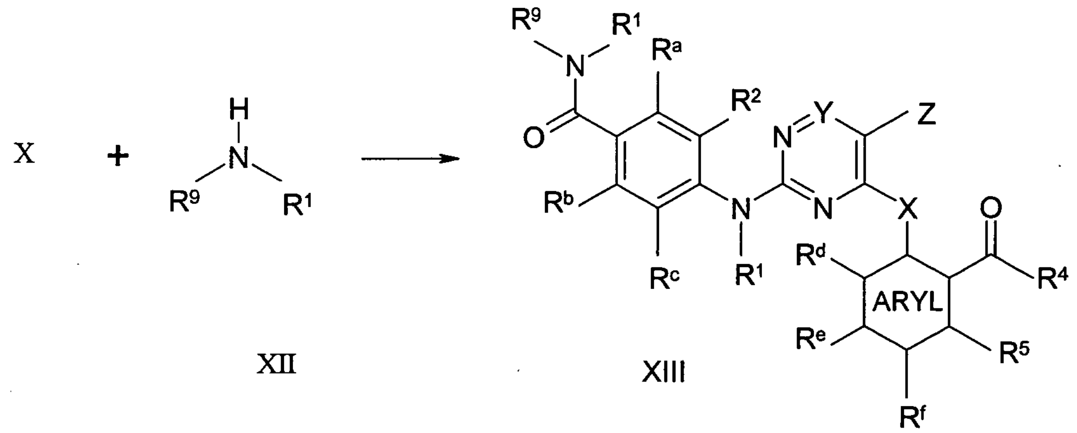

X

XII

XIII

oder:

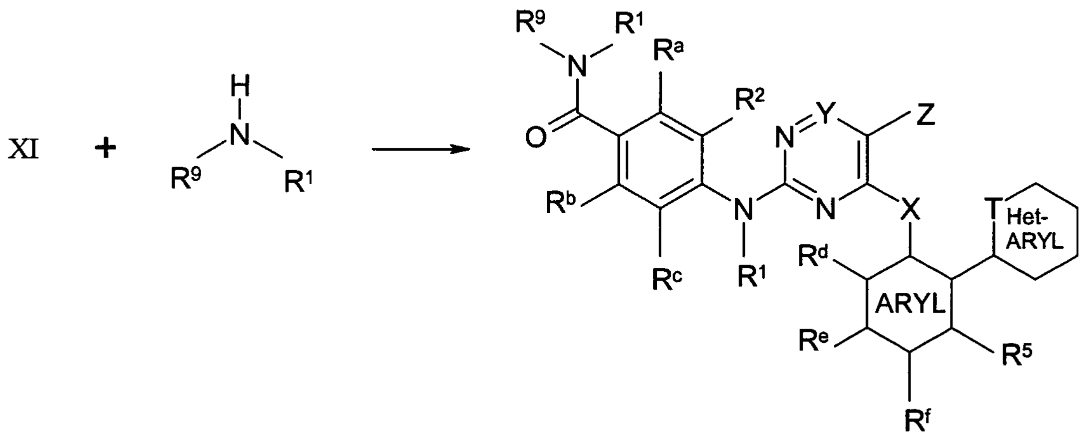

XI

XII

XIV

Es werden 1 Äquivalent der Verbindung **X** oder **XI,** 1 bis 1,5 Äquivalente der Verbindung **XII** und 1 bis 3 Äquivalente einer Base, beispielsweise Triethylamin oder Ethyldiisopropylamin in einem Lösungsmittel, beispielsweise 1,4-Dioxan, N,N-Dimethylformamid, N,N-Dimethylacetamid oder N-Methyl-2-pyrrolidinon gerührt. Bei einer Temperatur von 15 bis 25°C werden 1 bis 1,5 Äquivalente eines Kupplungsreagenzes, beispielsweise N,N-Dicyclohexylcarbodiimid, N,N-Diisopropylcarbodiimid, O-(Benzotriazol-1-yl)-N,N,N',N'-tetramethyluroniumtetrafluoroborat oder 1-(3-N,NDimethylaminopropyl)-3-ethylcarbodiimid zugegeben. Das Reaktionsgemisch wird 4 bis 24 h bei einer Temperatur von 15 bis 25°C weitergerührt. Danach wird das Lösungsmittel abdestilliert und der Rückstand chromatographisch gereinigt.

**Verfahren C**

**Stufe 1C**

Die Herstellung der Zwischenverbindung **XV** oder **XVI** erfolgt durch Substitution einer Abgangsgruppe LG, beispielsweise Halogen, SCN, Methoxy, vorzugsweise Chlor, an einem heteroaromatischem System **I** durch ein Nukleophiel **IV** oder **VI**.

## Schema 1C

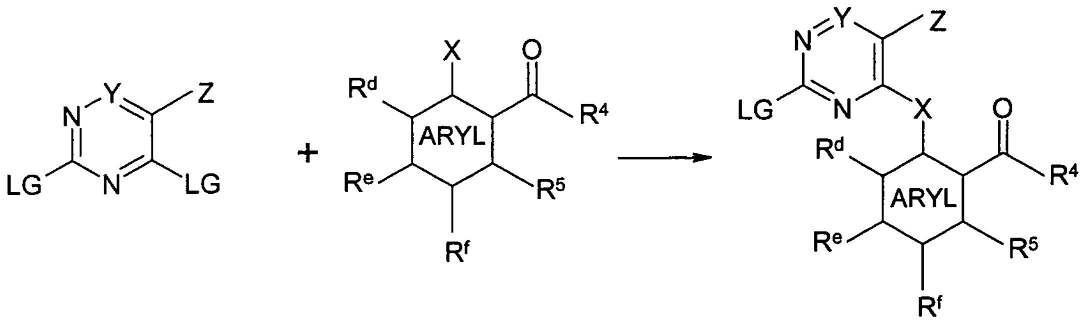

I IV XV

oder:

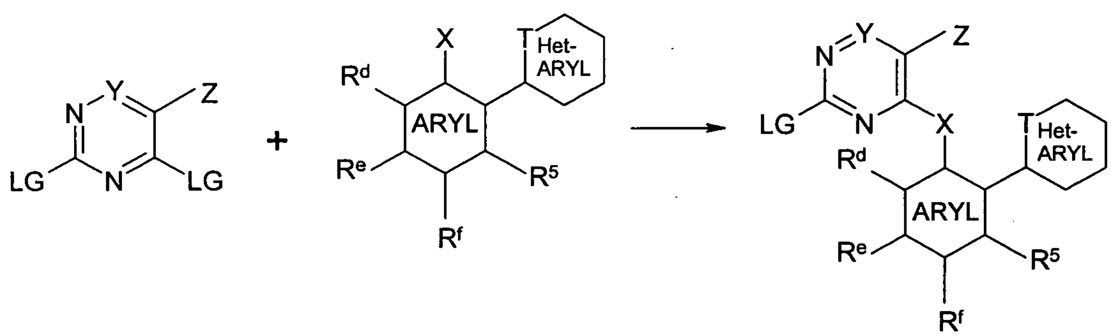

I VI XVI

Es werden 1 Äquivalent der Verbindung **I** und 1 bis 3 Äquivalente einer Base, beispielsweise Triethylamin oder Ethyldiisopropylamin einem Lösungsmittel, beispielsweise 1,4-Dioxan, Tetrahydrofuran, N,N-Dimethylformamid oder N,N-Dimethylacetamid gerührt. Bei einer Temperatur von -60 bis 0°C werden 0,8 bis 1,5 Äquivalente einer Verbindung **IV** oder **VI** zugegeben. Das Reaktionsgemisch wird 12 bis 72 h bei einer Temperatur von 15 bis 25°C weitergerührt. Danach wird das Lösungsmittel abdestilliert und der Rückstand chromatographisch gereinigt.

**Stufe 2C**

Die Herstellung der Endverbindung **V** oder **VII** erfolgt durch Substitution einer Abgangsgruppe LG, beispielsweise Halogen, SCN, Methoxy, vorzugsweise Chlor, an einem heteroaromatischem System **XV** oder **XVI** durch ein Nukleophil **II.**

**Schema 2C**

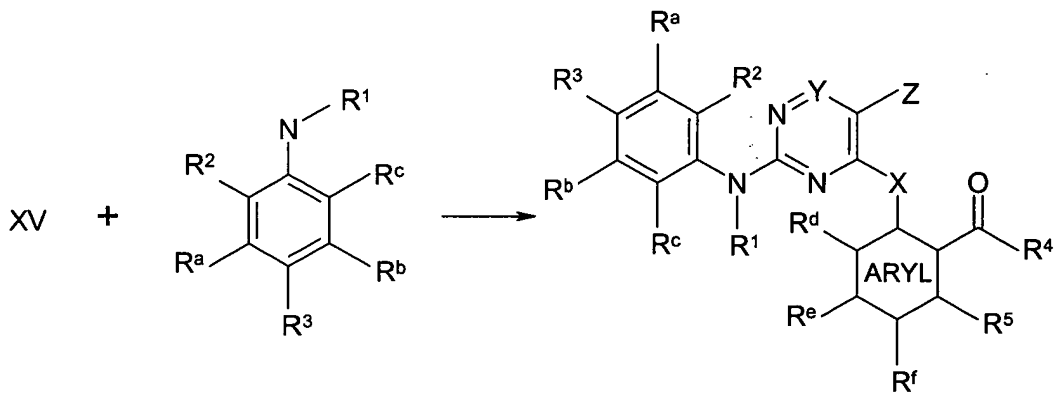


II V

oder:

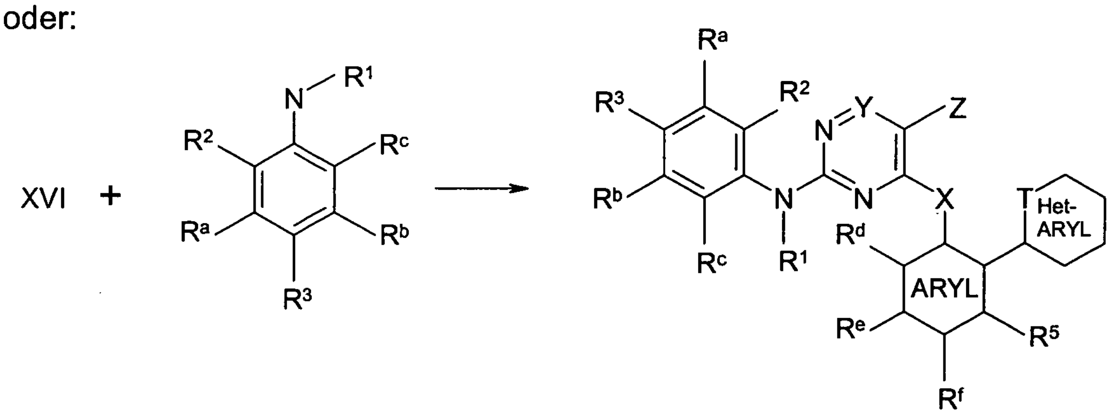


II VII

Es werden 1 Äquivalent der Verbindung **XV** oder **XVI** und 1 bis 1,5 Äquivalente der Verbindung **II** in einem Lösungsmittel, beispielsweise 1,4-Dioxan, N,N-Dimethylformamid, N,N-Dimethylacetamid oder N-Methyl-2-pyrrolidinon gerührt.

Bei einer Temperatur von 15 bis 40°C werden 1 bis 2 Äquivalente einer Säure, beispielsweise Schwefelsäure oder Salzsäure zugegeben. Das Reaktionsgemisch wird 12 bis 72 h bei einer Temperatur von 20 bis 100°C weitergerührt.

Danach wird das Lösungsmittel abdestilliert und der Rückstand chromatographisch gereinigt.

**Beispiel 1**

2-(2-Methoxy-4-N-propylcarbamoyl-phenylamino)-4-(2-carboxy-3-fluor-phenylamino)-5-trifluormethyl-pyrimidin

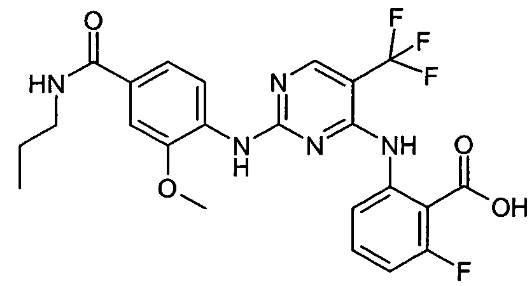

165 mg (0,424 mmol) 2-(2-Methoxy-4-propylcarbamoyl-phenylamino)-4-chlor-5-trifluormethyl-pyrimidin (Methode 1) werden in 400 µl 1,4-Dioxan gelöst und mit 72 mg (0,466 mmol) 2-Amino-6-fluor-benzoesäure versetzt. Diesem Reaktionsgemisch werden 106 µl einer 4 molaren Lösung von HCl (0,424 mmol) in 1,4-Dioxan zudosiert.
Nach einem Tag bei 50 °C wird das Lösungsmittel im Vakuum entfernt. Das Rohprodukt wird durch Säulenchromatographie gereinigt. Als Trägermaterial dient Kieselgel und als Eluent wird Dichlormethan, dem 18% einer Mischung aus 90% Methanol und 10% gesättigter wässriger Ammoniak-Lösung zugefügt sind, verwendet.

| | |
|---|---|
| Ausbeute | 212 mg (0,418 mmol; 98 %) eines weißen Feststoffs |
| UV max | 318 nm |
| MS (ESI) | 508 $(M+H)^+$ |
| $^1$H-NMR | 0.90 (t, 3H), 1.50 - 1.60 (m, 2H), 3.17 (s, 1H), 3.20 - 3.27 (m, 2H), 3.89 (s, 3H), 6.74 - 6.82 (m, 1H), 7.09 - 7.26 (m, 2H), 7.39 - 7.44 (m, 1H), 7.50 -7.53 (m, 1H), 7.98 - 8.06 (m, 2H), 8.36 - 8.41 (m, 2H), 8.48 (s, 1H) |

**Beispiele 2 - 10**

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes kommerziell erhältliches Anilin verwendet.
Als Lösungsmittel wird 1,4-Dioxan, N-Methyl-2-pyrrolidinon oder N,N-Dimethylacetamid verwendet.

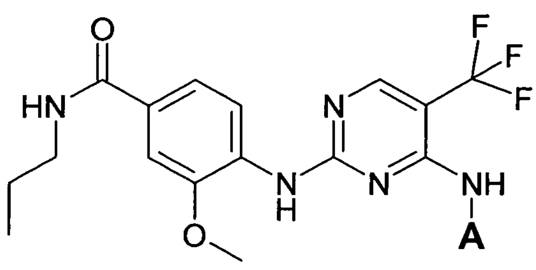

| # | A: | UV max [nm]: | MS (ESI) (M+H)$^+$: | NMR: |
|---|---|---|---|---|
| 2 | 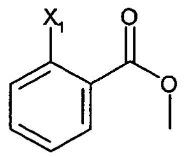 | 236/ 284 | 504 | 0.91 (t, 3H), 1.49 - 1.62 (m, 2H), 3.20 - 3.27 (m, 2H), 3.87 (d, 6H), 7.16 - 7.24 (m, 1H), 7.39 - 7.45 (m, 1H), 7.46 - 7.57 (m, 2H), 7.76 - 7.86 (m, 1H), 7.97 - 8.03 (m, 1H), 8.40 (t, 1H), 8.43 - 8.52 (m, 2H), 8.84 (s, 1H), 10.64 (s, 1H) |

| # | A: | UV max [nm]: | MS (ESI) (M+H)$^+$: | NMR: |
|---|---|---|---|---|
| 3 | 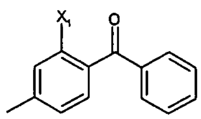 | 270 | 564 | 0.90 (t, 3H), 1.49 - 1.61 (m, 2H), 2.29 (s, 3H), 3.19 - 3.27 (m, 2H), 3.89 (s, 3H), 7.07 - 7.12 (m, 1H), 7.36 - 7.42 (m, 1H), 7.42 - 7.50 (m, 3H), 7.51 - 7.55 (m, 1H), 7.57 - 7.65 (m, 3H), 7.84 - 7.90 (m, 1H), 7.01 (s, 1H), 8.36 - 8.42 (m, 2H), 8.56 (s, 1H), 10.34 (s, 1H) |
| 4 | 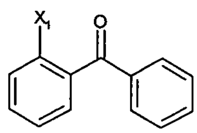 | 322 | 550 | 0.91 (t, 3H), 1.49 - 1.61 (m, 2H), 3.19 - 3.27 (m, 2H), 3.90 (s, 3H), 7.30 - 7.38 (m, 2H), 7.39 - 7.47 (m, 2H), 7.49 - 7.53 (m, 1H), 7.53 - 7.66 (m, 5H), 7.84 - 7.92 (m, 1H), 8.03 - 8.09 (m, 1H), 8.33 - 8.43 (m, 3H), 9.98 (s, 1H) |
| 5 | 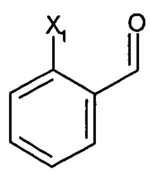 | 322 | 474 | 0.91 (t, 3H), 1.51 - 1.62 (m, 2H), 3.21 - 3.29 (m, 2H), 3.86 (s, 3H), 7.27 - 7.34 (m, 1H), 7.43 - 7.48 (m, 1H), 7.48 - 7.58 (m, 2H), 7.72 - 7.79 (m, 1H), 7.89 - 7.95 (m, 1H), 8.43 (t, 1H), 8.50 (s, 1H), 8.57 (s, 1H), 9.03 (s, 1H), 9.98 (s, 1H), 11.04 (s, 1H) |
| 6 | 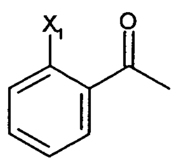 | 322 | 488 | 0.91 (t, 3H), 1.50 - 1.62 (m, 2H), 2.66 (s, 3H), 3.21 - 3.29 (m, 2H), 3.87 (s, 3H), 7.17 - 7.23 (m, 1H), 7.42 - 7.52 (m, 2H), 7.52 - 7.57 (m, 1H), 7.78 - 7.86 (m, 1H), 8.05 - 8.10 (m, 1H), 8.41 (t, 1H), 8.47 (s, 1H), 8.51 - 8.61 (m, 1H), 8.89 (s, 1H), 11.60 (s, 1H) |
| 7 | 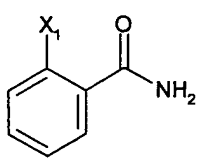 | 320 | 489 | 0.91 (t, 3H), 1.50 - 1.62 (m, 2H), 3.21 - 3.28 (m, 2H), 3.87 (s, 3H), 7.09 - 7.17 (m, 1H), 7.35 - 7.42 (m, 1H), 7.42 - 7.47 (m, 1H), 7.52 - 7.56 (m, 1H), 7.72 - 7.82 (m, 2H), 7.86 - 7.94 (m, 1H), 8.27 (s, 1H), 8.37 - 8.48 (m, 3H), 8.73 (s, 1H), 11.77 (s, 1H) |

| # | A: | UV max [nm]: | MS (ESI) $(M+H)^+$: | NMR: |
|---|---|---|---|---|
| 8 | 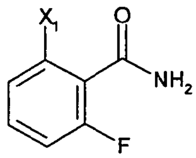 | 314 | 507 | 0.90 (t, 3H), 1.50 - 1.60 (m, 2H), 3.20 - 3.27 (m, 2H), 3.88 (s, 3H), 7.01 - 7.09 (m, 1H), 7.35 - 7.43 (m, 2H), 7.51 - 7.54 (m, 1H), 7.83 - 7.90 (m, 1H), 8.00 - 8.09 (m, 2H), 8.15 (s, 1H), 8.40 (t, 1H), 8.45 (s, 1H), 8.73 (s, 1H), 10.16 (s, 1H) |
| 9 | 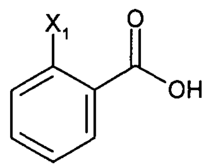 | 322 | 490 | (CDCl3) 1.00 (t, 3H), 1.60 - 1.72 (m, 2H), 1.92 (s, 1H), 3.38 - 3.48 (m, 2H), 3.97 (s, 3H), 6.05 - 6.15 (m, 1H), 7.11 - 7.22 (m, 2H), 7.46 (s, 1H), 7.52 - 7.62 (m, 1H), 7.99 (s, 1H), 8.11 - 8.21 (m, 1H), 8.35 - 8.45 (m, 2H), 8.56 - 8.64 (m, 1H), 10.98 (s, 1H) |
| 10 | 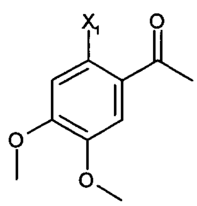 | 270 | 548 | 0.90 (t, 3H), 1.49 - 1.61 (m, 2H), 2.63 (s, 3H), 3.19 - 3.27 (m, 2H), 3.56 (s, 3H), 3.85 (s, 3H), 3.87 (s, 3H), 7.35 - 7.41 (m, 1H), 7.47 (s, 1H), 7.49 - 7.53 (m, 1H), 7.89 - 7.97 (m, 1H), 8.20 (s, 1H), 8.37 (t, 1H), 8.48 (s, 1H), 8.63 (s, 1H), 11.75 (s, 1H) |

**Beispiele 11 - 19**

Die folgenden Verbindungen sind über ein analoges Verfahren, wie in Beispiel 1 beschrieben, hergestellt. Die Herstellung des entsprechenden Anilins ist in Tetrahedron 2000, 56(37), 7245, Tetrahedron Letters 1992, 33(43), 6453, US 4,307,113 oder in Methode 2 - 5 beschrieben.
Als Lösungsmittel wird 1,4-Dioxan oder N,N-Dimethylacetamid verwendet.

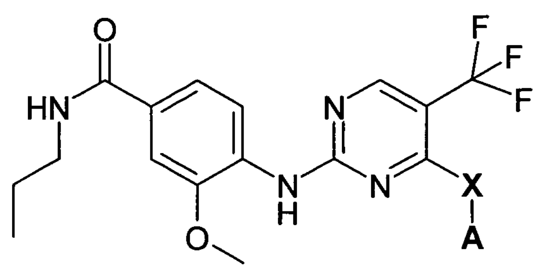

| # | X-A: | UV max [nm]: | MS (ESI) (M+H)$^+$: | NMR: |
|---|---|---|---|---|
| 11 | 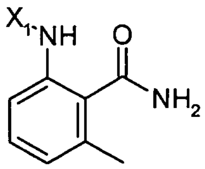 | 0 | 503 | 0.90 (t, 3H), 1.48 - 1.60 (m, 2H), 2.36 (s, 3H), 3.19 - 3.26 (m, 2H), 3.88 (s, 3H), 7.09 - 7.14 (m, 1H), 7.26 - 7.33 (m, 2H), 7.46 - 7.50 (m, 1H), 7.66 - 7.72 (m, 1H), 7.79 - 7.93 (m, 3H), 8.35 (t, 1H), 8.39 - 8.43 (m, 2H), 8.64 (s, 1H) |
| 12 | 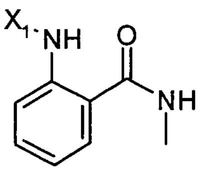 | 234 | 503 | 0.91 (t, 3H), 1.49 - 1.62 (m, 2H), 2.78 (d, 3H), 3.20 - 3.28 (m, 2H), 3.88 (s, 3H), 7.11 - 7.19 (m, 1H), 7.34 - 7.46 (m, 2H), 7.53 (s, 1H), 7.67 - 7.74 (m, 1H), 7.86 - 7.94 (m, 1H), 8.34 - 8.42 (m, 2H), 8.44 (s, 1H), 8.65 - 8.75 (m, 2H), 11.41 (s, 1H) |
| 13 | 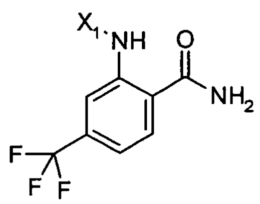 | 318 | 557 | 0.90 (t, 3H), 1.49 - 1.60 (m, 2H), 3.19 - 3.27 (m, 2H), 3.88 (s, 3H), 7.35 - 7.41 (m, 1H), 7.46 - 7.54 (m, 2H), 7.84 - 7.92 (m, 1H), 7.95 - 8.01 (m, 1H), 8.03 (s, 1H), 8.33 (t, 1H), 8.45 - 8.53 (m, 2H), 8.59 (s, 1H), 8.69 (s, 1H), 11.65 (s, 1H) |
| 14 | 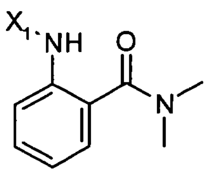 | 318 | 517 | 0.90 (t, 3H), 1.48 - 1.60 (m, 2H), 2.83 - 2.98 (m, 6H), 3.18 - 3.26 (m, 2H), 3.87 (s, 3H), 7.23 - 7.35 (m, 2H), 7.39 - 7.47 (m, 2H), 7.47 - 7.51 (m, 1H), 7.84 - 7.96 (m, 2H), 8.35 (t, 1H), 8.38 - 8.45 (m, 2H), 9.00 (s, 1H), |
| 15 | 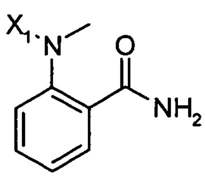 | 318 | 503 | 0.90 (t, 3H), 1.48 - 1.60 (m, 2H), 3.18 - 3.26 (m, 2H), 3.42 (s, 3H), 3.92 (s, 3H), 7.29 (s, 1H), 7.31 - 7.37 (m, 2H), 7.40 - 7.47 (m, 2H), 7.47 - 7.50 (m, 1H), 7.50 - 7.58 (m, 1H), 7.60 - 7.65 (m, 1H), 7.93 - 8.02 (m, 1H), 8.19 (s, 1H), 8.32 (t, 1H), 8.35 (s, 1H) |

| # | X-A: | UV max [nm]: | MS (ESI) $(M+H)^+$: | NMR: |
|---|---|---|---|---|
| 16 | 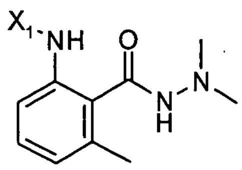 | 317 | 546 | 0.90 (t, 3H), 1.47 - 1.61 (m, 2H), 2.47 (s, 3H), 2.73 (s, 3H), 2.89 (s, 3H), 3.18 - 3.27 (m, 2H), 3.88 (s, 3H), 7.08 - 7.17 (m, 1H), 7.23 - 7.36 (m, 2H), 7.46 - 7.51 (m, 1H), 7.56 - 7.63 (m, 1H), 7.79 - 7.88 (m, 1H), 7.95 (s, 1H), 8.30 - 8.46 (m, 3H), 9.13 (s, 1H) |
| 17 | 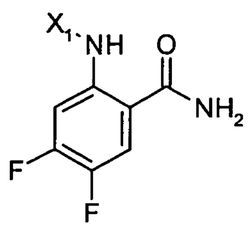 | 282 | 525 | 0.91 (t, 3H), 1.49 - 1.61 (m, 2H), 3.20 - 3.28 (m, 2H), 3.86 (s, 3H), 7.44 - 7.50 (m, 1H), 7.53 - 7.58 (m, 1H), 7.70 - 7.78 (m, 1H), 7.86 - 7.96 (m, 2H), 8.30 (s, 1H), 8.38 (t, 1H), 8.43 (s, 1H), 8.62 (s, 1H), 9.07 (s, 1H), 12.07 (s, 1H) |
| 18 | 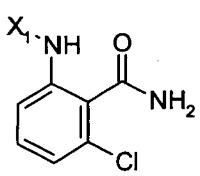 | 318 | 523 | |
| 19 | 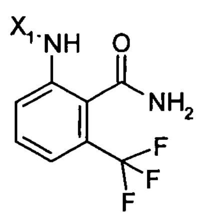 | 318 | 557 | 0.90 (t, 3H), 1.49 - 1.59 (m, 2H), 3.19 - 3.25 (m, 2H), 3.87 (s, 3H), 7.24 - 7.30 (m, 1H), 7.46 - 7.49 (m, 1H), 7.57 - 7.69 (m, 2H), 7.72 - 7.80 (m, 1H), 7.84 - 7.90 (m, 1H), 7.97 (s, 1H), 8.04 - 8.10 (m, 1H), 8.23 (s, 1H), 8.34 (t, 1H), 8.44 (s, 1H), 8.51 (s, 1H) |

**Beispiele 20 - 33**

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 6 beschrieben ist, verwendet.

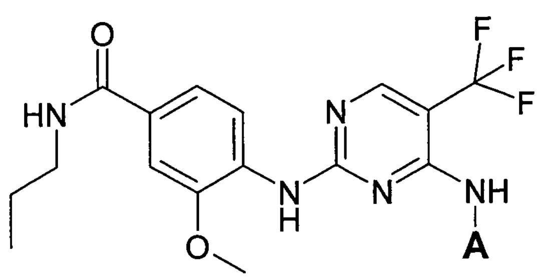

| # | A: | UV max [nm]: | MS (ESI) $(M+H)^+$: | NMR: |
|---|---|---|---|---|
| 20 | 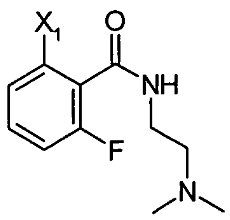 | 320 | 578 | 0.91 (t, 3H), 1.49 - 1.61 (m, 2H), 2.12 (s, 6H), 2.34 (t, 2H), 3.20 - 3.28 (m, 2H), 3.88 (s, 3H), 7.05 - 7.12 (m, 1H), 7.35 - 7.45 (m, 2H), 7.49 - 7.53 (m, 1H), 7.81 - 7.87 (m, 1H), 7.90 - 7.97 (m, 1H), 8.38 (t, 1H), 8.44 (s, 1H), 8.49 (s, 1H), 8.67 (s, 1H), 9.65 (s, 1H) |
| 21 | 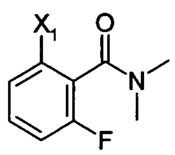 | 275 | 535 | 0.90 (t, 3H), 1.48 - 1.60 (m, 2H), 2.80 (s, 3H), 2.93 (s, 3H), 3.18 - 3.26 (m, 2H), 3.87 (s, 3H), 7.16 - 7.24 (m, 1H), 7.24 - 7.32 (m, 1H), 7.44 - 7.53 (m, 2H), 7.65 - 7.74 (m, 1H), 7.75 - 7.84 (m, 1H), 8.35 (t, 1H), 8.41 (s, 1H), 8.45 (s, 1H), 8.64 (s, 1H) |
| 22 | 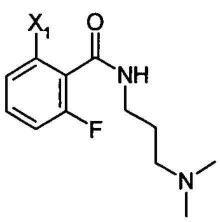 | 320 | 592 | 0.91 (t, 3H), 1.50 - 1.64 (m, 4H), 2.09 (s, 6H), 2.22 (t, 2H), 3.20 - 3.28 (m, 4H), 3.87 (s, 3H), 7.05 - 7.11 (m, 1H), 7.36 - 7.43 (m, 2H), 7.50 - 7.53 (m, 1H), 7.82 - 7.89 (m, 1H), 7.91 - 7.98 (m, 1H), 8.39 (t, 1H), 8.44 (s, 1H), 8.66 - 8.73 (m, 2H), 9.65 (s, 1H) |
| 23 | 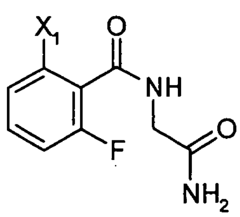 | 315 | 546 | 0.91 (t, 3H), 1.50 - 1.60 (m, 2H), 3.20 - 3.27 (m, 2H), 3.84 (d, 2H), 3.88 (s, 3H), 7.02 - 7.13 (m, 2H), 7.33 (s, 1H), 7.36 - 7.46 (m, 2H), 7.50 - 7.53 (m, 1H), 7.81 - 7.90 (m, 1H), 7.96 - 8.05 (m, 1H), 8.38 (t, 1H), 8.44 (s, 1H), 8.61 - 8.70 (m, 2H), 9.99 (s, 1H) |

| # | A: | UV max [nm]: | MS (ESI) $(M+H)^+$: | NMR: |
|---|---|---|---|---|
| 24 | 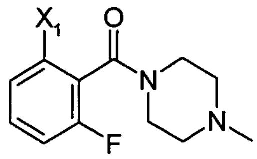 | 320 | 591 | 0.90 (t, 3H), 1.49 - 1.59 (m, 2H), 1.68 - 1.80 (m, 1H), 1.95 - 2.04 (m, 1H), 2.06 (s, 3H), 2.10 - 2.20 (m, 1H), 2.37 - 2.46 (m, 1H), 3.08 - 3.15 (m, 2H), 3.19 - 3.26 (m, 2H), 3.33 - 3.43 (m, 1H), 3.69 - 3.80 (m, 1H), 3.87 (s, 3H), 7.18 - 7.28 (m, 2H), 7.44 - 7.48 (m, 1H), 7.48 - 7.55 (m, 1H), 7.58 - 7.76 (m, 2H), 8.34 (t, 1H), 8.38 - 8.48 (m, 2H), 8.62 (s, 1H) |
| 25 | 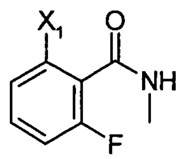 | 315 | 521 | 0.91 (t, 3H), 1.50 - 1.60 (m, 2H), 2.77 (d, 3H), 3.20 - 3.27 (m, 2H), 3.88 (s, 3H), 7.04 - 7.11 (m, 1H), 7.36 - 7.44 (m, 2H), 7.49 - 7.54 (m, 1H), 7.82 - 7.90 (m, 1H), 7.93 - 8.01 (m,1 H), 8.38 (t, 1H), 8.44 (s, 1H), 8.53 - 8.62 (m, 1H), 8.69 (s, 1H), 9.77 (s, 1H) |
| 26 | 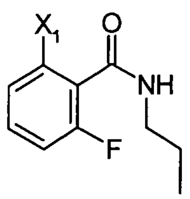 | 318 | 549 | 0.85 (t, 3H), 0.91 (t, 3H), 1.43 - 1.60 (m, 4H), 3.16 - 3.27 (m, 4H), 3.88 (s, 3H), 7.01 - 7.12 (m, 1H), 7.35 - 7.43 (m, 2H), 7.49 - 7.54 (m, 1H), 7.81 - 7.89 (m, 1H), 7.91 - 7.99 (m, 1H), 8.36 - 8.47 (m, 2H), 8.59 - 8.74 (m, 2H), 9.56 (s, 1H) |
| 27 | 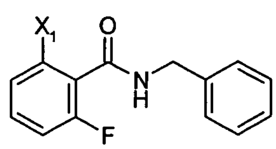 | 315 | 597 | 0.90 (t, 3H), 1.52 - 1.58 (m, 2H), 3.20 - 3.26 (m, 2H), 3.87 (s, 3H), 4.47 (d, 2H), 7.07 - 7.14 (m, 1H), 7.20 - 7.26 (m, 1H), 7.26 - 7.31 (m, 4H), 7.36 - 7.44 (m, 2H), 7.49 - 7.52 (m, 1H), 7.82 - 7.87 (m, 1H), 7.93 - 7.99 (m, 1H), 8.38 (t, 1H), 8.44 (s, 1H), 8.69 (s, 1H), 9.13 - 9.20 (m, 1H), 9.61 (s, 1H) |
| 28 | 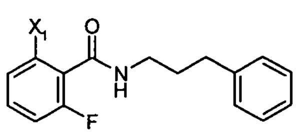 | 315 | 625 | 0.90 (t, 3H), 1.51 - 1.60 (m, 2H), 1.72 - 1.81 (m, 2H), 2.58 (t, 2H), 3.21 - 3.28 (m, 4H), 3.87 (s, 3H), 7.06 - 7.13 (m, 1H), 7.13 - 7.19 (m, 3H), 7.22 - 7.28 (m, 2H), 7.36 - 7.44 (m, 2H), 7.50 - 7.53 (m, 1H), 7.83 - 7.88 (m, 1H), 7.91 - 7.97 (m, 1H), 8.38 (t, 1H), 8.43 (s, 1H), 8.67 - 8.73 (m, 2H), 9.55 (s, 1H) |

| # | A: | UV max [nm]: | MS (ESI) $(M+H)^+$: | NMR: |
|---|---|---|---|---|
| 29 | 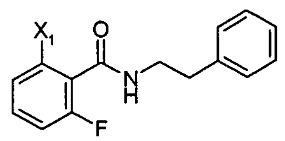 | 318 | 612 | 0.91 (t, 3H), 1.51 - 1.58 (m, 2H), 2.79 (t, 2H), 3.21 - 3.27 (m, 2H), 3.44 - 3.50 (m, 2H), 3.88 (s, 3H), 7.04 - 7.10 (m, 1H), 7.11 - 7.25 (m, 5H), 7.36 - 7.42 (m, 2H), 7.50 - 7.53 (m, 1H), 7.82 - 7.89 (m, 1H), 7.89 - 7.97 (m, 1H), 8.38 (t, 1H), 8.43 - 8.46 (m, 1H), 8.67 - 8.70 (m, 1H), 8.71 - 8.77 (m, 1H), 9.57 (s, 1H) |
| 30 | 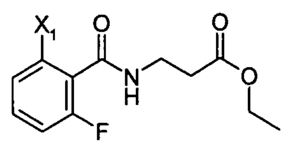 | 315 | 607 | 0.91 (t, 3H), 1.14 (t, 3H), 1.50 - 1.60 (m, 2H), 2.53 (t, 2H), 3.21 - 3.27 (m, 2H), 3.43 - 3.50 (m, 2H), 3.88 (s, 3H), 4.03 (q, 2H), 7.05 - 7.11 (m, 1H), 7.36 - 7.44 (m, 2H), 7.52 (s, 1H), 7.82 - 7.89 (m, 1H), 7.91 - 7.98 (m, 1H), 8.38 (t, 1H), 8.44 (s, 1H), 8.68 (s, 1H), 8.70 - 8.75 (m, 1H), 9.62 (s, 1H) |
| 31 | 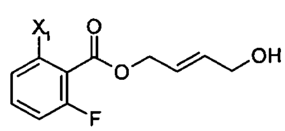 | 318 | 578 | 0.90 (t, 3H), 1.49 - 1.59 (m, 2H), 3.19 - 3.26 (m, 2H), 3.88 (s, 3H), 3.97 - 4.03 (m, 2H), 4.71 - 4.79 (m, 3H), 5.36 - 5.44 (m, 1H), 5.62 - 5.70 (m, 1H), 7.16 - 7.24 (m, 1H), 7.25 - 7.31 (m, 1H), 7.46 - 7.50 (m, 1H), 7.53 - 7.60 (m, 1H), 7.68 (m, 1H), 7.78 - 7.86 (m, 1H), 8.35 (t, 1H), 8.39 (s, 1H), 8.44 (s, 1H), 9.55 (s, 1H) |
| 32 | 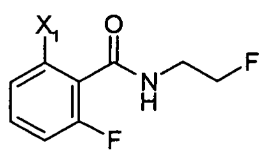 | 316 | 553 | 0.91 (t, 3H), 1.49 - 1.61 (m, 2H), 3.19 - 3.27 (m, 2H), 3.48 - 3.55 (m, 1H), 3.55 - 3.61 (m, 1H), 3.88 (s, 3H), 4.43 (t, 1H), 4.55 (t, 1H), 7.05 - 7.13 (m, 1H), 7.35 - 7.45 (m, 2H), 7.50 - 7.53 (m, 1H), 7.81 - 7.89 (m, 1H), 7.92 - 7.99 (m, 1H), 8.37 (t, 1H), 8.44 (s, 1H), 8.69 (s, 1H), 8.87 (t, 1H), 9.55 (s, 1H) |
| 33 | 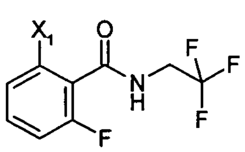 | 230 | 589 | 0.90 (t, 3H), 1.50 - 1.60 (m, 2H), 3.20 - 3.27 (m, 2H), 3.88 (s, 3H), 4.02 - 4.13 (m, 2H), 7.10 - 7.17 (m, 1H), 7.35 - 7.40 (m, 1H), 7.42 - 7.49 (m, 1H), 7.49 - 7.53 (m, 1H), 7.80 - 7.94 (m, 2H), 8.38 (t, 1H), 8.44 (s, 1H), 8.68 (s, 1H), 9.23 - 9.30 (m, 2H) |

**Beispiele 34 - 41**

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 7 beschrieben ist, verwendet.

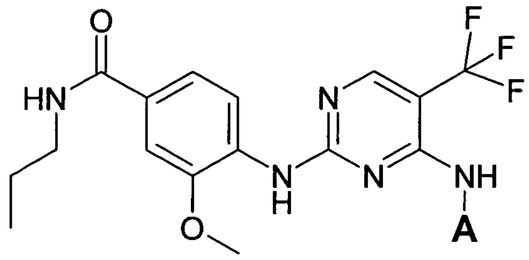

| # | A: | UV max [nm]: | MS (ESI) $(M+H)^+$: | NMR: |
|---|---|---|---|---|
| 34 | 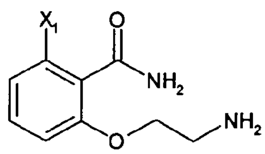 | 282/318 | 548 | 0.91 (t, 3H), 1.50 - 1.60 (m, 2H), 2.98 - 3.05 (m, 2H), 3.20 - 3.27 (m, 2H), 3.89 (s, 3H), 4.15 (t, 2H), 6.86 - 6.93 (m, 1H), 7.29 - 7.35 (m, 1H), 7.37 - 7.43 (m, 1H), 7.49 - 7.54 (m, 1H), 7.79 - 7.87 (m, 1H), 7.90 - 7.98 (m, 2H), 8.23 (s, 1H), 8.29 (s, 1H), 8.39 (t, 1H), 8.42 (s, 1H), 8.58 (s, 1H), 10.65 (s, 1H), |
| 35 | 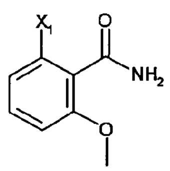 | 314 | 519 | 0.91 (t, 3H), 1.50 - 1.60 (m, 2H), 3.20 - 3.27 (m, 2H), 3.85 (s, 3H), 3.88 (s, 3H), 6.87 - 6.91 (m, 1H), 7.30 - 7.36 (m, 1H), 7.36 - 7.41 (m, 1H), 7.50 - 7.53 (m, 1H), 7.77 - 7.84 (m, 2H), 7.91 - 7.97 (m, 2H), 8.38 (t, 1H), 8.42 (s, 1H), 8.57 (s, 1H), 10.55 (s, 1H) |
| 36 | 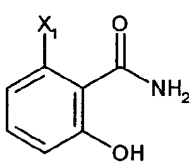 | 314 | 505 | 0.90 (t, 3H), 1.49 - 1.61 (m, 2H), 3.19 - 3.27 (m, 2H), 3.88 (s, 3H), 6.60 - 6.74 (m, 1H), 7.12 - 7.22 (m, 1H), 7.35 - 7.44 (m, 1H), 7.48 - 7.54 (m, 1H), 7.67 - 8.10 (m, 3H), 8.31 - 8.45 (m, 3H), 8.45 - 8.65 (m, 1H), 11.85 (s, 1H) |

| # | A: | UV max [nm]: | MS (ESI) $(M+H)^+$: | NMR: |
|---|---|---|---|---|
| 37 | 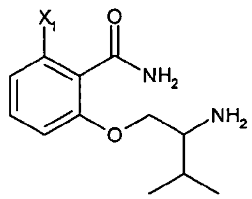 | 230/282 /318 | 590 | 0.86 - 0.99 (m, 9H), 1.50 - 1.61 (m, 2H), 1.72 - 1.84 (m, 1H), 2.90 - 2.97 (m, 1H), 3.20 - 3.27 (m, 2H), 3.89 (s, 3H), 3.90 - 3.96 (m, 1H), 4.16 - 4.22 (m, 1H), 6.90 - 6.95 (m, 1H), 7.28 - 7.36 (m, 1H), 7.36 - 7.42 (m, 1H), 7.49 - 7.54 (m, 1H), 7.77 - 7.87 (m, 1H), 7.90 - 7.98 (m, 2H), 8.20 - 8.24 (m, 1H), 8.33 (s, 1H), 8.39 (t, 1H), 8.42 (s, 1H), 8.57 (s, 1H), 10.66 (s, 1H) |
| 38 | 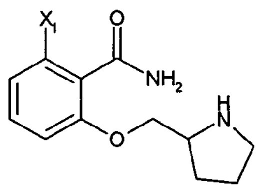 | 230/282 /318 | 588 | 0.91 (t, 3H), 1.50 - 1.61 (m, 3H), 1.62 - 1.73 (m, 1H), 1.73 - 1.83 (m, 1H), 1.83 - 1.93 (m, 1H), 2.86 - 2.92 (m, 2H), 3.20 - 3.27 (m, 2H), 3.52 - 3.59 (m, 1H), 3.89 (s, 3H), 3.93 - 4.00 (m, 1H), 4.06 - 4.13 (m, 1H), 6.86 - 6.92 (m, 1H), 7.27 - 7.35 (m, 1H), 7.35 - 7.43 (m, 1H), 7.48 - 7.55 (m, 1H), 7.80 - 7.89 (m, 1H), 7.90 - 8.00 (m, 2H), 8.25 - 8.31 (m, 2H), 8.39 (t, 1H), 8.42 (s, 1H), 8.59 (s, 1H), 10.78 (s, 1H) |
| 39 | 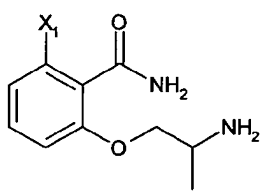 | 282/318 | 562 | 0.91 (t, 3H), 1.12 (d, 3H), 1.50 - 1.60 (m, 2H), 3.19 - 3.27 (m, 2H), 3.83 - 3.91 (m, 4H), 4.00 - 4.06 (m, 1H), 6.85 - 6.92 (m, 1H), 7.27 - 7.35 (m, 1H), 7.35 - 7.42 (m, 1H), 7.49 - 7.54 (m, 1H), 7.77 - 7.86 (m, 1H), 7.90 - 8.00 (m, 2H), 8.24 - 8.32 (m, 2H), 8.38 (t, 1H), 8.42 (s, 1H), 8.57 (s, 1H), 10.63 (s, 1H) |
| 40 | 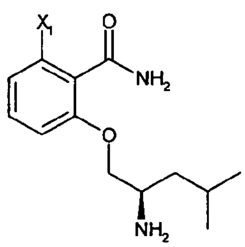 | 226/282 /318 | 604 | 0.84 - 0.94 (m, 9H), 1.21 - 1.36 (m, 2H), 1.50 - 1.60 (m, 2H), 1.72 - 1.82 (m, 1H), 3.08 - 3.16 (m, 1H), 3.20 - 3.27 (m, 2H), 3.80 - 3.87 (m, 1H), 3.89 (s, 3H), 4.04 - 4.10 (m, 1H), 6.86 - 6.91 (m, 1H), 7.27 - 7.35 (m, 1H), 7.36 - 7.42 (m, 1H), 7.49 - 7.54 (m, 1H), 7.77 - 7.87 (m, 1H), 7.90 - 7.98 (m, 2H), 8.28 (s, 1H), 8.34 (s, 1H), 8.38 (t, 1H), 8.42 (s, 1H), 8.57 (s, 1H), 10.72 (s, 1H) |

| # | A: | UV max [nm]: | MS (ESI) (M+H)+: | NMR: |
|---|---|---|---|---|
| 41 | 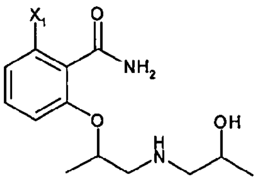 | 226/282 /318 | 620 | 0.91 (t, 3H), 0.95 - 1.05 (m, 3H), 1.08 - 1.35 (m, 3H), 1.50 - 1.60 (m, 2H), 2.38 - 2.45 (m, 2H), 2.69 - 2.80 (m, 2H), 3.19 - 3.28 (m, 2H), 3.57 - 3.69 (m, 1H), 3.89 (s, 3H), 4.53 - 4.65 (m, 1H), 6.85 - 6.94 (m, 1H), 7.26 - 7.35 (m, 1H), 7.37 - 7.44 (m, 1H), 7.49 - 7.55 (m, 1H), 7.79 - 7.91 (m, 2H), 7.91 - 7.98 (m, 1H), 8.23 (s, 1H), 8.37 - 8.44 (m, 2H), 8.58 - 8.63 (m, 1H), 10.79 (s, 1H) |

## Beispiele 42 - 45

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 6 beschrieben ist, verwendet.

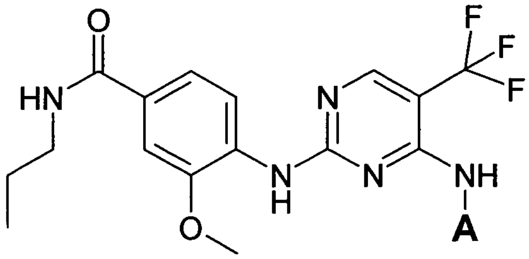

| # | A: | UV max [nm]: | MS (ESI) $(M+H)^+$: | NMR: |
|---|---|---|---|---|
| 42 | 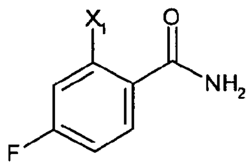 | 315 | 507 | 0.91 (t, 3H), 1.49 - 1.61 (m, 2H), 3.20 - 3.28 (m, 2H), 3.88 (s, 3H), 6.89 - 6.98 (m, 1H), 7.43 - 7.49 (m, 1H), 7.53 - 7.57 (m, 1H), 7.74 - 7.82 (m, 2H), 7.83 - 7.91 (m, 1H), 8.27 (s, 1H), 8.33 - 8.47 (m, 3H), 8.99 (s, 1H), 12.26 (s, 1H) |

| # | A: | UV max [nm]: | MS (ESI) $(M+H)^+$: | NMR: |
|---|---|---|---|---|
| 43 | 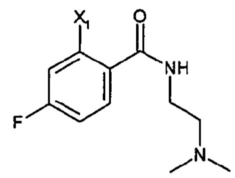 | 285/320 | 578 | 0.91 (t, 3H), 1.50 - 1.60 (m, 2H), 2.17 (s, 6H), 2.41 (t, 2H), 3.20 - 3.27 (m, 2H), 3.87 (s, 3H), 6.92 - 7.00 (m, 1H), 7.42 - 7.47 (m, 1H), 7.52 - 7.56 (m, 1H), 7.73 - 7.84 (m, 2H), 8.26 - 8.41 (m, 2H), 8.44 (s, 1H), 8.68 (s, 1H), 8.96 (s, 1H), 11.67 (s, 1H) |
| 44 | 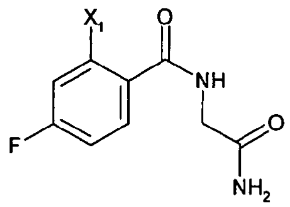 | 230/285 /320 | 564 | |
| 45 | 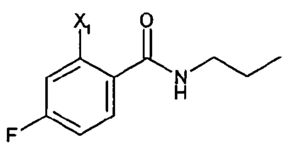 | 318 | 549 | 0.85 - 0.94 (m, 6H), 1.49 - 1.60 (m, 4H), 3.18 - 3.27 (m, 4H), 3.87 (s, 3H), 6.92 - 7.00 (m, 1H), 7.42 - 7.49 (m, 1H), 7.55 (s, 1H), 7.75 - 7.84 (m, 2H), 8.28 - 8.41 (m, 2H), 8.44 (s, 1H), 8.73 - 8.80 (m, 1H), 8.96 (s, 1H), 11.71 (s, 1H) |

<u>**Beispiele 46 - 48**</u>

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 8 beschrieben ist, verwendet.

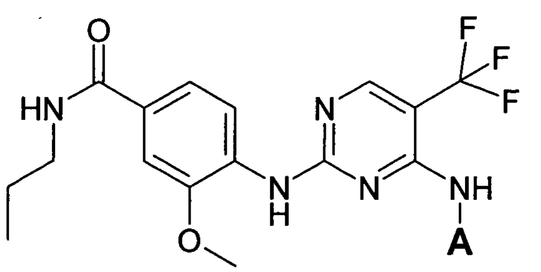

| # | A: | UV max [nm]: | MS (ESI) $(M+H)^+$: | NMR: |
|---|---|---|---|---|
| 46 | 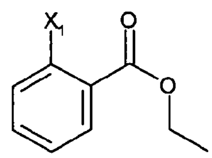 | 238/ 286 | 518 | 0.91 (t, 3H), 1.29 (t, 3H), 1.51 - 1.60 (m, 2H), 3.21 - 3.27 (m, 2H), 3.87 (s, 3H), 4.31 (q, 2H), 7.18 - 7.23 (m, 1H), 7.39 - 7.44 (m, 1H), 7.45 - 7.56 (m, 2H), 7.77 - 7.84 (m, 1H), 7.98 - 8.02 (m, 1H), 8.37 - 8.49 (m, 3H), 8.81 (s, 1H), 10.66 (s, 1H) |
| 47 | 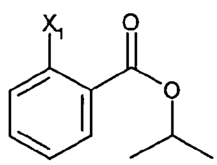 | 230/ 282/ 306 | 532 | 0.91 (t, 3H), 1.28 (d, 6H), 1.50 - 1.60 (m, 2H), 3.21 - 3.27 (m, 2H), 3.87 (s, 3H), 5.14 (sept, 1H), 7.18 - 7.24 (m, 1H), 7.37 - 7.42 (m, 1H), 7.47 - 7.55 (m, 2H), 7.77 - 7.84 (m, 1H), 7.95 - 8.00 (m, 1H), 8.34 - 8.44 (m, 2H), 8.47 (s, 1H), 8.77 (s, 1H), 10.63 (s, 1H) |
| 48 | 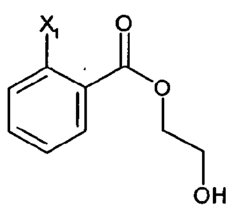 | 282/ 318 | 534 | (CDCl3) 1.00 (t, 3H), 1.47 - 1.72 (m, 3H), 3.39 - 3.47 (m, 2H), 3.95 - 4.01 (m, 5H), 4.48 - 4.54 (m, 2H), 6.05 - 6.14 (m, 1H), 7.12 - 7.19 (m, 2H), 7.42 - 7.47 (m, 1H), 7.53 - 7.61 (m, 1H), 7.91 (s, 1H), 8.08 - 8.14 (m, 1H), 8.37 - 8.46 (m, 2H), 8.52 - 8.59 (m, 1H), 10.73 (s, 1H) |

**Beispiele 49 - 52**

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 9 beschrieben ist, verwendet.

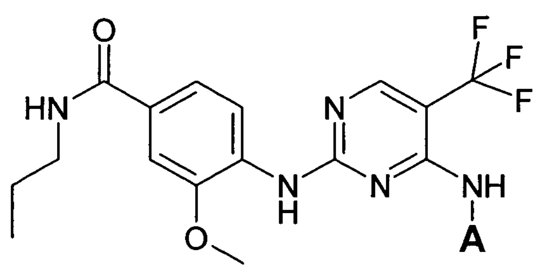

| # | A: | UV max [nm]: | MS (ESI) $(M+H)^+$: | NMR: |
|---|---|---|---|---|
| 49 | 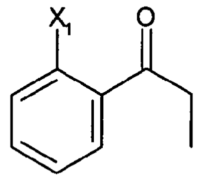 | 280 | 502 | 0.91 (t, 3H), 1.07 (t, 3H), 1.50 - 1.62 (m, 2H), 3.11 (q, 2H), 3.21 - 3.27 (m, 2H), 3.87 (s, 3H), 7.17 - 7.24 (m, 1H), 7.40 - 7.51 (m, 2H), 7.52 - 7.56 (m, 1H), 7.79 - 7.86 (m, 1H), 8.05 - 8.11 (m, 1H), 8.41 (t, 1H), 8.45 - 8.55 (m, 2H), 8.83 (s, 1H), 11.49 (s, 1H) |
| 50 | 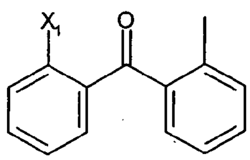 | 230 - 330 | 564 | 0.91 (t, 3H), 1.50 - 1.62 (m, 2H), 2.20 (s, 3H), 3.20 - 3.28 (m, 2H), 3.89 (s, 3H), 7.17 - 7.28 (m, 3H), 7.28 - 7.34 (m, 1H), 7.37 - 7.44 (m, 3H), 7.51 - 7.59 (m, 2H), 7.79 - 7.89 (m, 1H), 8.32 - 8.46 (m, 3H), 8.75 (s, 1H), 10.86 (s, 1H) |
| 51 | 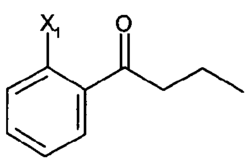 | 230 | 516 | 0.86 - 0.94 (m, 6H), 1.49 - 1.67 (m, 4H), 3.05 (t, 2H), 3.20 - 3.29 (m, 2H), 3.87 (s, 3H), 7.17 - 7.24 (m, 1H), 7.40 - 7.51 (m, 2H), 7.52 - 7.56 (m, 1H), 7.77 - 7.86 (m, 1H), 8.05 - 8.11 (m, 1H), 8.40 - 8.55 (m, 3H), 8.88 (s, 1H), 11.47 (s, 1H) |
| 52 | 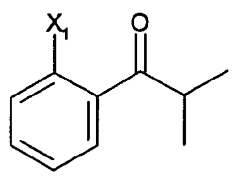 | 270 | 516 | 0.91 (t, 3H), 1.10 (d, 6H), 1.49 - 1.62 (m, 2H), 3.21 - 3.28 (m, 2H), 3.67 - 3.79 (m, 1H), 3.87 (s, 3H), 7.19 - 7.26 (m, 1H), 7.40 - 7.45 (m, 1H), 7.45 - 7.52 (m, 1H), 7.52 - 7.56 (m, 1H), 7.77 - 7.86 (m, 1H), 8.06 - 8.13 (m, 1H), 8.41 - 8.53 (m, 3H), 8.87 (s, 1H), 11.40 (s, 1H) |

## Beispiele 53 - 56

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches kommerziell erhältlich oder in Journal of the Chemical Society, Perkin Transactions 1: 1979, (9), 2203 oder in Journal of Medicinal Chemistry 1963, 6(5), 471-80 beschrieben ist, verwendet.

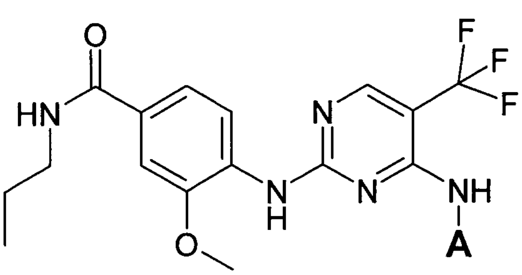

| # | A: | UV max [nm]: | MS (ESI) $(M+H)^+$: | NMR: |
|---|---|---|---|---|
| 53 | 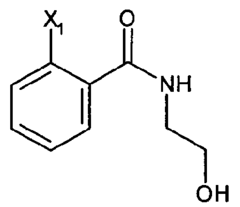 | 266 | 533 | 0.91 (t, 3H), 1.51 - 1.61 (m, 2H), 3.20 - 3.28 (m, 2H), 3.47 - 3.55 (m, 2H), 3.88 (s, 3H), 4.73 (t, 1H), 7.12 - 7.19 (m, 1H), 7.36 - 7.45 (m, 2H), 7.51 - 7.55 (m, 1H), 7.71 - 7.77 (m, 1H), 7.86 - 7.94 (m, 1H), 8.32 - 8.45 (m, 3H), 8.66 - 8.74 (m, 2H), 11.27 (s, 1H) |
| 54 | 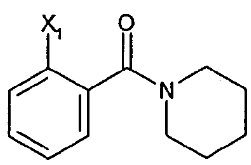 | 316 | 557 | 0.90 (t, 3H), 1.12 - 1.33 (m, 2H), 1.36 - 1.41 (m, 2H), 1.41 - 1.60 (m, 4H), 3.17 - 3.27 (m, 4H), 3.48 - 3.59 (m, 2H), 3.87 (s, 3H), 7.23 - 7.33 (m, 2H), 7.33 - 7.38 (m, 1H), 7.41 - 7.51 (m, 2H), 7.75 - 7.85 (m, 1H), 7.85 - 7.95 (m, 1H), 8.36 (t, 1H), 8.40 (s, 1H), 8.43 (s, 1H), 8.80 (s, 1H) |
| 55 | 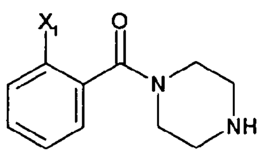 | 313 | 556 (M-H) | 0.90 (t, 3H), 1.49 - 1.60 (m, 2H), 2.28 - 2.42 (m, 2H), 2.56 - 2.68 (m, 2H), 3.07 - 3.19 (m, 2H), 3.19 - 3.27 (m, 2H), 3.87 (s, 3H), 7.21 - 7.28 (m, 1H), 7.30 - 7.38 (m, 2H), 7.43 - 7.51 (m, 2H), 7.74 - 7.81 (m, 1H), 7.81 - 7.88 (m, 1H), 8.34 - 8.44 (m, 3H), 8.83 (s, 1H) |
| 56 | 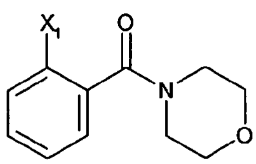 | 241, 250 | 559 | 0.90 (t, 3H), 1.49 - 1.59 (m, 2H), 3.18 - 3.26 (m, 2H), 3.45 - 3.65 (m, 4H), 3.87 (s, 3H), 7.21 - 7.27 (m, 1H), 7.30 - 7.36 (m, 1H), 7.38 - 7.42 (m, 1H), 7.45 - 7.50 (m, 2H), 7.73 - 7.81 (m, 1H), 7.81 - 7.86 (m, 1H), 8.34 (t, 1H), 8.38 (s, 1H), 8.40 (s, 1H) |

**Beispiel 57**

2-[2-(2-Chloro-phenoxy)-4-*N*-propylcarbamoyl-phenylamino]-4-(2-acetyl-phenylamino)-5-trifluormethyl-pyrimidin

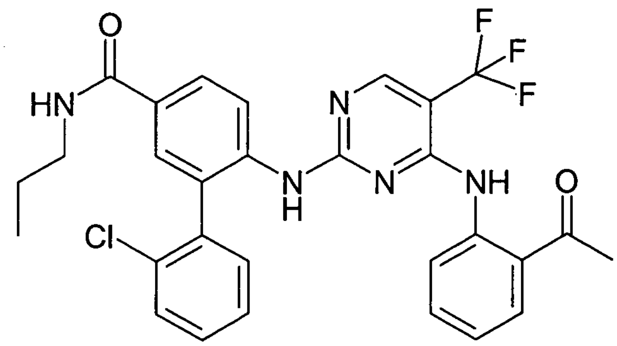

50 mg (0,16 mmol) 4-(2-Acetyl-phenylamino)-2-chlor-5-trifluormethyl-pyrimidin (Methode 10) werden in 0,2 ml 1,4-Dioxan gelöst, mit 60 mg (0,17 mmol) 4-Amino-3-(2-chlor-phenoxy)-*N*-propyl-benzamid hydrochlorid (Methode 11) versetzt und 3 Tage bei 50 °C gerührt. Anschließend wird das Lösungsmittel im Vakuum entfernt und das Rohprodukt durch Säulenchromatographie gereinigt, wobei als Trägermaterial C18 RP-Gel verwendet wird.

Das Produkt wird mit einem Gradient eluiert, dessen Startbedingungen aus Wasser : Acetonitril = 95% : 5% bestehen und der sich innerhalb von 20 min auf die Endbedingungen Wasser : Acetonitril = 5% : 95% ändert.

| | |
|---|---|
| Ausbeute | 64 mg (0,088 mmol; 55 %) eines gelben Feststoffs |
| MS (ESI) | 584/586 $(M+H)^+$ Isomerenmuster $^{35}Cl$ / $^{37}Cl$ |
| UV max | 282 nm |
| $^1H$-NMR | 0.88 (t, 3H), 1.47 - 1.57 (m, 2H), 2.66 (s, 3H), 3.17 - 3.23 (m, 2H), 6.87 -6.94 (m, 1H), 7.12 - 7.20 (m, 2H), 7.23 - 7.30 (m, 1H), 7.36 - 7.44 (m, 2H), 7.52 - 7.56 (m, 1H), 7.67 - 7.72 (m, 1H), 7.77 - 7.85 (m, 1H), 8.04 - 8.10 (m, 1H), 8.41 - 8.49 (m, 2H), 8.55 - 8.63 (m, 1H), 9.37 (s, 1H), 11.63 (s, 1H) |

**Beispiele 58 - 63**

Die folgenden Verbindungen sind über ein analoges Verfahren, wie in Beispiel 57 beschrieben, hergestellt. Dabei wird 4-(2-Acetyl-phenylamino)-2-chlor-5-trifluormethylpyrimidin und ein entsprechendes Anilin, welches in Methode 11 beschrieben ist, verwendet.

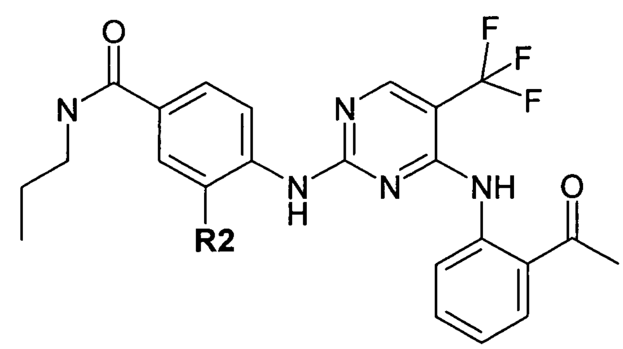

| # | R2: | UV max [nm]: | MS (ESI) (M+H)$^+$: | NMR: |
|---|---|---|---|---|
| 58 | 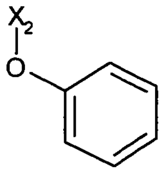 | 278 | 550 | 0.87 (t, 3H), 1.47 - 1.56 (m, 2H), 2.66 (s, 3H), 3.16 - 3.23 (m, 2H), 6.91 - 6.96 (m, 2H), 7.08 - 7.13 (m, 1H), 7.16 - 7.21 (m, 1H), 7.30 - 7.36 (m, 2H), 7.39 - 7.48 (m, 2H), 7.64 - 7.69 (m, 1H), 7.83 - 7.88 (m, 1H), 8.05 - 8.10 (m, 1H), 8.41 - 8.46 (m, 2H), 8.56 - 8.63 (m, 1H), 9.31 (s, 1H), 11.64 (s, 1H) |
| 59 | 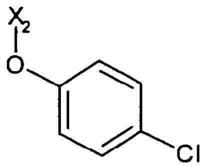 | 282 | 584 | 0.88 (t, 3H), 1.47 - 1.57 (m, 2H), 2.66 (s, 3H), 3.17 - 3.23 (m, 2H), 6.92 - 6.98 (m, 2H), 7.15 - 7.22 (m, 1H), 7.33 - 7.38 (m, 2H), 7.39 - 7.46 (m, 1H), 7.48 - 7.52 (m, 1H), 7.67 - 7.72 (m, 1H), 7.82 - 7.87 (m, 1H), 8.05 - 8.10 (m, 1H), 8.41 - 8.48 (m, 2H), 8.54 - 8.62 (m, 1H), 9.39 (s, 1H), 11.64 (s, 1H) |
| 60 | 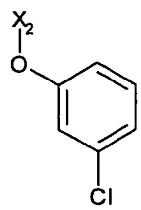 | 243 | 584 | 0.89 (t, 3H), 1.48 - 1.58 (m, 2H), 2.66 (s, 3H), 3.18 - 3.25 (m, 2H), 6.87 - 6.93 (m, 2H), 7.10 - 7.15 (m, 1H), 7.15 - 7.20 (m, 1H), 7.29 - 7.35 (m, 1H), 7.35 - 7.43 (m, 1H), 7.54 - 7.58 (m, 1H), 7.72 - 7.77 (m, 1H), 7.81 - 7.86 (m, 1H), 8.04 - 8.10 (m, 1H), 8.41 (s, 1H), 8.48 (t, 1H), 8.51 - 8.59 (m, 1H), 9.43 (s, 1H), 11.66 (s, 1H) |

| # | R2: | UV max [nm]: | MS (ESI) (M+H)$^+$: | NMR: |
|---|---|---|---|---|
| 61 | 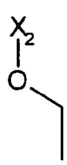 | 318 | 502 | 0.91 (t, 3H), 1.32 (t, 3H), 1.51 - 1.60 (m, 2H), 2.65 (s, 3H), 3.20 - 3.27 (m, 2H), 4.13 (q, 2H), 7.18 - 7.24 (m, 1H), 7.40 - 7.51 (m, 2H), 7.51 - 7.55 (m, 1H), 7.80 - 7.88 (m, 1H), 8.05 - 8.10 (m, 1H), 8.40 (t, 1H), 8.46 - 8.57 (m, 2H), 8.79 (s, 1H), 11.55 (s, 1H) |
| 62 | $X_2$—Br | 286 | 536 | 0.91 (t, 3H), 1.51 - 1.61 (m, 2H), 2.65 (s, 3H), 3.22 - 3.29 (m, 2H), 7.10 - 7.17 (m, 1H), 7.24 - 7.33 (m, 1H), 7.66 - 7.72 (m, 1H), 7.86 - 7.92 (m, 1H), 8.02 - 8.08 (m, 1H), 8.18 - 8.22 (m, 1H), 8.39 - 8.49 (m, 2H), 8.57 (t, 1H), 9.59 (s, 1H), 11.73 (s, 1H) |

**Beispiel 63**

2-[2-Methoxy-4-(4-morpholin-4-yl-(1,4-trans-cyclohexyl)carbamoyl)-phenylamino]-4-2-carbamoyl-3-fluor-phenylamino)-5-trifluormethyl-pyrimidin

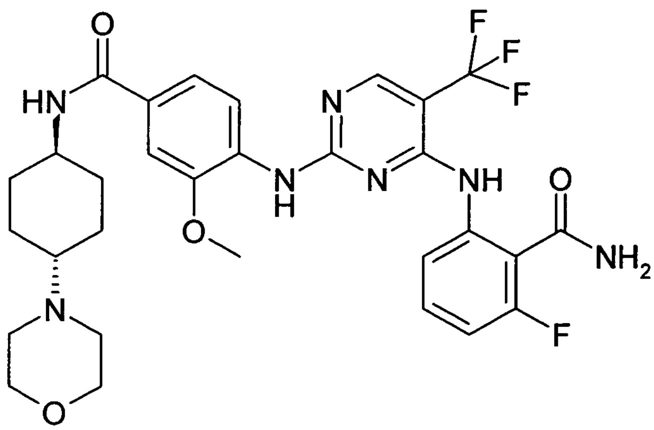

200 mg (0,49 mmol) 2-(4-Carboxyamino-2-methoxy-phenylamino)-4-chlor-5-trifluormethyl-pyrimidin (Methode 12) werden in 0,5 ml *N*-Methyl-2-pyrrolidinon gelöst und mit 83mg(0,54 mmol) 2-Amino-6-fluor-benzamid versetzt. Diesem Reaktionsgemisch werden 120 µl einer 4 M Lösung von HCl (0,49 mmol) in 1,4-Dioxan zudosiert. Nach 16 h bei 90°C wird das Reaktionsgemisch in 150 ml einer wässrigen 1 N Salzsäure eingerührt. Der Niederschlag wird abfiltriert und im Vakuum getrocknet. 50 mg (0,11 mmol) dieses Niederschlages, 94 µl (0,54 mmol) *N*-Ethyldiisopropylamin, 45 mg (0,13 mmol) TBTU und 30 mg (0,16 mmol) 4-(4-Amino-cyclohexyl)-morpholin (Methode 13) werden in 0,4 ml Tetrahydrofuran gelöst.
Nach 15h bei Raumtemperatur wird das Lösungsmittel im Vakuum entfernt. Das Rohprodukt wird durch Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet.

| | |
|---|---|
| Ausbeute | 43 mg (0,068 mmol; 62 %) eines hellgelben Feststoffs |
| MS (ESI) | 633 $(M+H)^+$ |
| $^1$H-NMR | 1.20 - 1.45 (m, 4H), 1.82 - 1.97 (m, 4H), 2.14 - 2:24 (m, 1H), 3.52 - 3.60 (m, 4H), 3.66 - 3.80 (m, 1H), 3.88 (s, 3H), 7.01 - 7.10 (m, 1H), 7.33 - 7.43 (m, 2H), 7.47 - 7.53 (m, 1H), 7.82 - 7.90 (m, 1H), 7.98 - 8.08 (m, 2H), 8.08 - 8.18 (m, 2H), 8.44 (s, 1H), 8.71 (s, 1H), 10.16 (s, 1H) |

**Beispiele 64 - 71**

Die folgenden Verbindungen sind über ein analoges Verfahren, wie in Beispiel 63 beschrieben, hergestellt. Das entsprechende Anilin ist in Methode 6, 14 oder 15 beschrieben. Das für die Darstellung des Amids eingesetzte Amin ist kommerziell erhältlich oder ist in Methode 13 beschrieben.

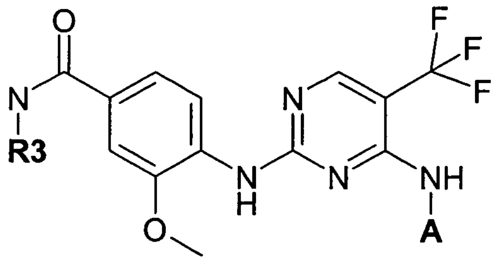

| # | A | R3 | UV max [nm]: | MS (ESI) (M+H)$^+$: | NMR: |
|---|---|---|---|---|---|
| 64 | 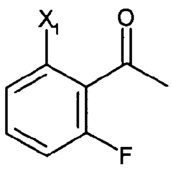 | 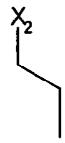 | 284, 246 | 506 | 0.91 (t, 3H), 1.50 – 1.61 (m, 2H), 2.51 - 2.56 (m, 3H), 3.20 - 3.28 (m, 2H), 3.88 (s, 3H), 7.10 - 7.20 (m, 1H), 7.30 - 7.40 (m, 1H), 7.47 - 7.57 (m, 2H), 7.74 - 7.85 (m, 1H), 7.90 - 8.05 (m, 1H), 8.40 (t, 1H), 8.46 (s, 1H), 8.68 (s, 1H), 10.45 (s, 1H) |
| 65 | 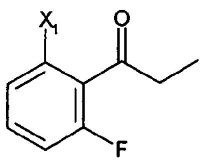 | 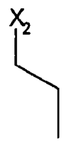 | 243 | 520 | 0.90 (t, 3H), 0.97 (t, 3H), 1.49 – 1.60 (m, 2H), 2.81 - 2.90 (m, 2H), 3.19 - 3.26 (m, 2H), 3.87 (s, 3H), 7.15 - 7.24 (m, 1H), 7.26 - 7.32 (m, 1H), 7.47 - 7.57 (m, 2H), 7.70 - 7.83 (m, 2H), 8.37 (t, 1H), 8.43 (s, 1H), 8.55 (s, 1H), 9.98 (s, 1H) |
| 66 | 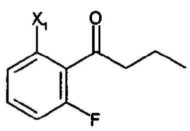 | 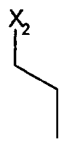 | 319 | 534 | 0.77 (t, 3H), 0.91 (t, 3H), 1.44 – 1.61 (m, 4H), 2.76 - 2.85 (m, 2H), 3.18 - 3.27 (m, 2H), 3.88 (s, 3H), 7.15 - 7.25 (m, 1H), 7.25 - 7.33 (m, 1H), 7.46 - 7.58 (m, 2H), 7.76 (t, 2H), 8.36 (t, 1H), 8.43 (s, 1H), 9.51 (s, 1H), 8.87 (s, 1H) |
| 67 | 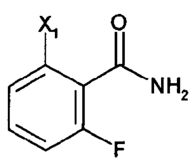 | 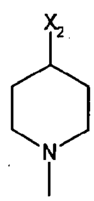 | 320 | 562 | 1.53 - 1.67 (m, 2H), 1.72 - 1.82 (m, 2H), 1.90 - 2.00 (m, 2H), 2.17 (s, 3H), 2.74 - 2.83 (m, 2H), 3.68 - 3.81 (m, 1H), 3.88 (s, 3H), 7.01 - 7.10 (m, 1H), 7.34 - 7.44 (m, 2H), 7.49 - 7.53 (m, 1H), 7.84 - 7.90 (m, 1H), 8.00 - 8.09 (m, 2H), 8.10 - 8.19 (m, 2H), 8.45 (s, 1H), 8.71 (s, 1H), 10.16 (s, 1H) |
| 68 | 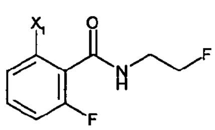 | 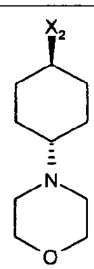 | 318 | 678 | 1.23 - 1.44 (m, 4H), 1.83 - 1.96 (m, 4H), 2.14 - 2.24 (m, 1H), 3.49 - 3.54 (m, 1H), 3.54 - 3.62 (m, 5H), 3.67 - 3.78 (m, 1H), 3.88 (s, 3H), 4.41 - 4.47 (m, 1H), 4.51 - 4.56 (m, 1H), 7.06 - 7.14 (m, 1H), 7.34 - 7.45 (m, 2H), 7.47 - 7.53 (m, 1H), 7.79 - 7.90 (m, 1H), 7.90 - 8.02 (m, 1H), 8.08 - 8.16 (m, 1H), 8.44 (s, 1H), 8.70 (s, 1H), 8.89 (t, 1H), 9.57 (s, 1H) |

| # | A | R3 | UV max [nm]: | MS (ESI) $(M+H)^+$: | NMR: |
|---|---|---|---|---|---|
| 69 | 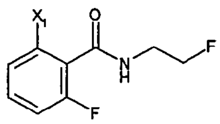 | 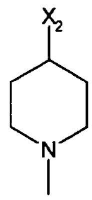 | 318 | 608 | 1.54 - 1.67 (m, 2H), 1.74 - 1.82 (m, 2H), 1.90 - 2.00 (m, 2H), 2.18 (s, 3H), 2.75 - 2.83 (m, 2H), 3.49 - 3.55 (m, 1H), 3.55 - 3.60 (m, 1H), 3.68 - 3.80 (m, 1H), 3.88 (s, 3H), 4.41 - 4.46 (m, 1H), 4.51 - 4.56 (m, 1H), 7.06 - 7.14 (m, 1H), 7.36 - 7.45 (m, 2H), 7.48 - 7.52 (m, 1H), 7.80 - 7.90 (m, 1H), 7.91 - 8.02 (m, 1H), 8.11 - 8.19 (m, 1H), 8.44 (s, 1H), 8.71 (s, 1H), 8.89 (t, 1H), 9.57 (s, 1H) |
| 70 | 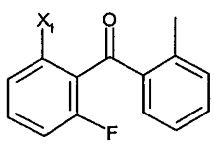 | 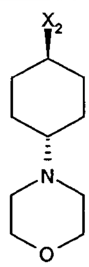 | 314 | 707 | 1.22 - 1.43 (m, 4H), 1.83 - 1.96 (m, 4H), 2.15 - 2.22 (m, 1H), 2.32 (s, 3H), 3.53 - 3.58 (m, 4H), 3.67 - 3.78 (m, 1H), 3.92 (s, 3H), 6.91 - 7.00 (m, 1H), 7.16 - 7.28 (m, 3H), 7.28 - 7.37 (m, 2H), 7.44 - 7.49 (m, 1H), 7.50 - 7.58 (m, 1H), 7.61 - 7.69 (m, 1H), 7.73 - 7.86 (m, 1H), 8.05 - 8.11 (m, 1H), 8.16 (s, 1H), 8.24 (s, 1H), 9.17 (s, 1H) |
| 71 | 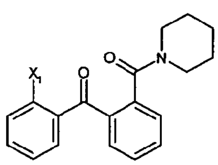 | 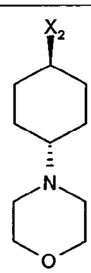 | 318 | 786 | 1.22 - 1.55 (m, 10H), 1.83 - 1.96 (m, 4H), 2.15 - 2.24 (m, 1H), 3.53 - 3.59 (m, 4H), 3.69 - 3.79 (m, 1H), 3.89 (s, 3H), 7.14 - 7.21 (m, 1H), 7.38 - 7.45 (m, 3H), 7.48 - 7.57 (m, 4H), 7.58 - 7.64 (m, 1H), 7.81 - 7.88 (m, 1H), 8.11 - 8.17 (m, 1H), 8.36 - 8.52 (m, 4H), 8.79 (s, 1H), 10.74 (s, 1H) |

**Methode 1**

2-(2-Methoxy-4-propylcarbamoyl-phenylamino)-4-chlor-5-trifluormethyl-pyrimidin

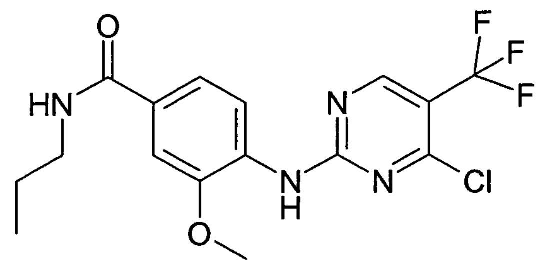

5,00 g (21,9 mmol) 2,4-Dichlor-5-trifluormethyl-pyrimidin werden in 50 ml 1,4-Dioxan gelöst und mit 5,50 g (21,9 mmol) 4-Amino-3-methoxybenzoesäure-propylamid Hydrochlorid (analog Journal of Pharmaceutical Sciences 1989, 78(10), 829-32) versetzt. Diesem Reaktionsgemisch werden 7,50 ml (43,8 mmol) Ethyldiisopropylamin zugesetzt und 2 Tage bei Raumtemperatur gerührt. Anschließend wird das Reaktionsgemisch mit 250 ml Ethylacetat verdünnt und zunächst mit 300 ml wässriger 10%-iger $KHSO_4$-Lsg., dann mit 300 ml gesättigter, wässriger NaCl-Lösung gewaschen. Die organische Phase wird mit $MgSO_4$ getrocknet und das Lösungsmittel im Vakuum entfernt.
Das Rohprodukt wird durch Säulenchromatographie gereinigt. Als Trägermaterial dient Kieselgel und als Eluent wird ein Gemisch bestehend aus Cyclohexan : Ethylacetat (75:25) verwendet.

| | |
|---|---|
| Ausbeute | 2,30 g (5,9 mmol; 27 %) |
| $^1$H-NMR | 0.91 (t, 3H), 1.50 - 1.61 (m, 2H), 3.20 - 3.28 (m, 2H), 3.87 (s, 3H), 7.46 -7.51 (m, 1H), 7.52 - 7.56 (m, 1H), 7.70 - 7.75 (m, 1H), 8.44 (t, 1H), 8.75 (s, 1H), 9.73 (s, 1H) |

**Methode 2**

2-Amino-6-methyl-benzamid

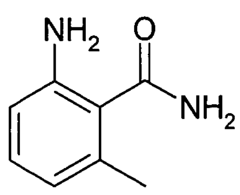

5,25 g (28,1 mmol) 2-Methyl-6-nitrobenzoesäure werden zu 250 ml Thionylchlorid gegeben und unter Rückfluss 3 h erhitzt. Anschließend wird das Thionylchlorid im Vakuum entfernt. Vom dem Rückstand werden 2,93 g (14,7 mmol) in 50 ml THF gelöst, auf 0 °C heruntergekühlt und mit 44 ml einer wässrigen, 32%-igen Ammoniak-Lösung versetzt. Dieses Reaktionsgemisch wird über Nacht unter Rühren auf Raumtemperatur erwärmt. Anschließend wird das Reaktionsgemisch mit 100 ml Ethylacetat verdünnt und dreimal mit je 50 ml einer gesättigten, wässrigen NaCl-Lösung gewaschen. Die organische Phase wird mit $MgSO_4$ getrocknet und das Lösungsmittel im Vakuum entfernt. 2,44 g (13,5 mmol) dieses Reaktionsproduktes werden in 100 ml THF gelöst und mit 200 mg Pd auf Kohle (10% Pd) versetzt. Das Reaktionsgemisch wird 16 h bei 3 bar $H_2$-Druck und Raumtemperatur hydriert. Danach wird der Katalysator abfiltriert und das Lösungsmittel im Vakuum entfernt.

| | |
|---|---|
| Ausbeute | 2,03 g (13,5 mmol; 48%) |

(fortgesetzt)

| | |
|---|---|
| $^1$H-NMR | 2,21 (s, 3H), 4.88 (s, 2H), 6.36 - 6.42 (m, 1H), 6.48 - 6.55 (m, 1H), 6.87 - 6.96 (m, 1H), 7.38 (s, 1H), 7.61 (s, 1H) |

Analog zu dieser Methode werden folgende Verbindungen hergestellt:

| | MS (ESI) (M+H)$^+$: |
|---|---|
| 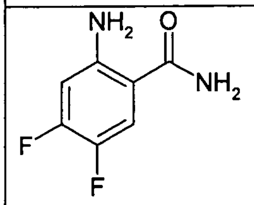 | 173 |
| 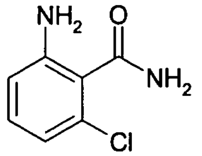 | 171 |

**Methode 3:**

2-Methylamino-benzamid

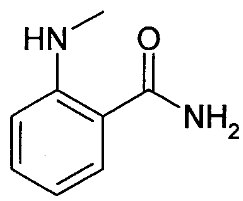

2,0 g (16,5 mmol) 2-Fluorbenzonitril werden mit 10 ml (80,0 mmol) einer 8 M Methylamin-Lösung in Ethanol versetzt und 20 h bei 100 °C in einem Druckgefäß erhitzt. Anschließend wird das Reaktionsgemisch mit 200 ml Ethylacetat verdünnt und dreimal mit je 160 ml einer wässrigen 10%-igen NaCl-Lösung gewaschen. Die organische Phase wird mit $MgSO_4$ getrocknet und das Lösungsmittel im Vakuum entfernt. 1,0 g (7,6 mmol) dieses Reaktionsproduktes werden mit 20 ml einer 20%-igen wässrigen Schwefelsäure versetzt und 3 h bei 80 °C gerührt. Anschließend wird das Reaktionsgemisch mit 200 ml Ethylacetat verdünnt und dreimal mit je 160 ml einer wässrigen 10%-igen NaCl-Lösung gewaschen. Danach wird die organische Phase mit $MgSO_4$ getrocknet, das Lösungsmittel im Vakuum entfernt.
Das Rohprodukt wird durch Säulenchromatographie gereinigt. Als Trägermaterial dient Kieselgel und als Eluent wird Dichlormethan, dem 15% einer Mischung aus 90%
Methanol und 10% gesättigter wässriger Ammoniak-Lösung zugefügt sind, verwendet.

| | |
|---|---|
| Ausbeute | 777 mg (5,2 mmol; 32%) |
| MS (ESI) | 151 (M+H)$^+$ |
| $^1$H-NMR | 2.78 (d, 3H), 6.49 - 6.56 (m, 1H), 6.59 - 6.66 (m, 1H), 7.09 (s, 1H), 7.24 - 7.3 3 (m, 1H), 7.55 - 7.63 (m, 1H), 7.77 (s, 1H), 7.92 - 8.05 (m, 1H) |

**Methode 4:**

2-Amino-6-methyl-benzoesäure-*N',N'*-dimethyl-hydrazid

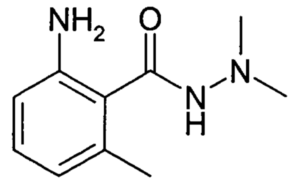

2-Dimethylamino-7-nitro-2,3-dihydro-isoindol-1-on ist analog zu den Verbindungen, die in Journal of the Chemical Society of Pakistan 1985, 7(1), 69-70, Patent W0 0314315 und Patent US 5716993 beschrieben sind, hergestellt.
240 mg (1,1 mmol) 2-Dimethylamino-7-nitro-2,3-dihydro-isoindol-1-on werden in 70 ml Dimethylformamid und 50 ml Methanol gelöst und mit 100 mg Pd auf Kohle (10% Pd) versetzt. Das Reaktionsgemisch wird 16 h bei 3 bar $H_2$-Druck und Raumtemperatur hydriert. Anschließend wird der Katalysator abfiltriert und das Lösungsmittel im Vakuum entfernt.

| | |
|---|---|
| Ausbeute | 204 mg (1,0 mmol; 91%) |
| MS (ESI) | 194 $(M+H)^+$ |

**Methode 5**

2-Amino-6-trifluormethyl-benzamid

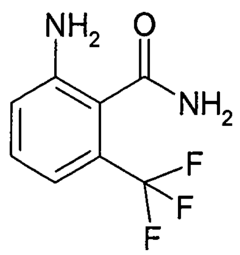

100 mg (0,5 mmol) 2-Fluor-6-trifluormethyl-benzonitril werden mit 1 ml einer 7 M Ammoniak-Lösung in Methanol versetzt und 20 min. bei 100 °C in der Mikrowelle erhitzt. Anschließend wird das Lösungsmittel im Vakuum entfernt, das Rohprodukt mit 1 ml konz. $H_2SO_4$ versetzt und 2,5 h bei 80 °C erhitzt. Danach wird das Reaktionsgemisch in Eiswasser eingerührt, mit 1 M NaOH neutralisiert und zweimal mit je 150 ml Dichlormethan und dreimal mit je 160 ml Ethylacetat extrahiert. Die organische Phase wird mit $MgSO_4$ getrocknet, das Lösungsmittel im Vakuum entfernt.
Das Rohprodukt wird durch Säulenchromatographie gereinigt. Als Trägermaterial dient Kieselgel und als Eluent wird Dichlormethan, dem 4% einer Mischung aus 90% Methanol und 10% gesättigter wässriger Ammoniak-Lösung zugefügt sind, verwendet.

| | |
|---|---|
| Ausbeute | 31 mg (0,152mmo1; 30 %) |
| MS (ESI) | 205 $(M+H)^+$ |

**Methode 6:**

2-Amino-*N*-(2-dimethylamino-ethyl)-6-fluor-benzamid

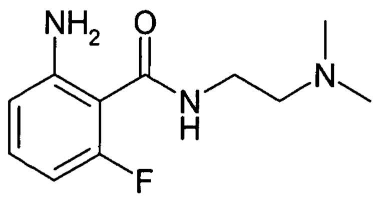

500 mg (3,2 mmol) 2-Amino-6-fluorbenzoesäure, 284 µl (3,2 mmol) 2-N,N-Dimethylaminoethylamin und 563 µl (3,2 mmol) Diisopropylethylamin werden in 2 ml Tetrahydrofuran gelöst und mit 1,08 ml (3,2 mmol) TBTU versetzt. Diese Reaktionsmischung wird 2,5 h bei Raumtemperatur gerührt. Anschließend wird die Reaktionsmischung mit 100 ml 10 %iger wässrigen Kaliumhydrogencarbonat-Lösung versetzt und sechsmal mit je 100 ml Ethylacetat extrahiert. Danach wird die organische Phase mit $MgSO_4$ getrocknet, das Lösungsmittel im Vakuum entfernt.
Das Rohprodukt wird durch Säulenchromatographie gereinigt. Als Trägermaterial dient Kieselgel und als Eluent wird Dichlormethan, dem 8% einer Mischung aus 90% Methanol und 10% gesättigter wässriger Ammoniak-Lösung zugefügt sind, verwendet.

| | |
|---|---|
| Ausbeute | 154 mg (21 %) |
| MS (ESI) | 226 $(M+H)^+$ |

Analog zu dieser Methode werden folgende Verbindungen hergestellt. Dabei wird 2-Amino-6-fluorbenzoesäure und ein entsprechendes kommerziell erhältliches Amin verwendet.

| | **MS (ESI) $(M+H)^+$:** |
|---|---|
| 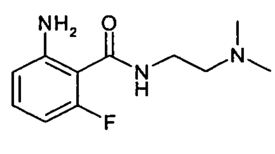 | 226 |
| 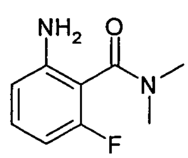 | 183 |
| 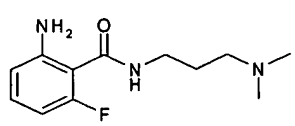 | 240 |

| | |
|---|---|
| 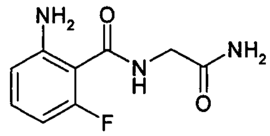 | 212 |
| 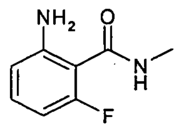 | 169 |
| 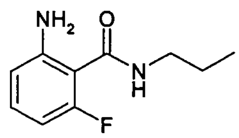 | 197 |
| 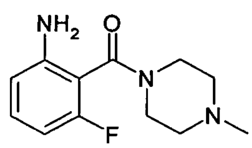 | 238 |
| 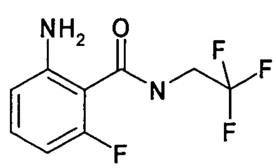 | 237 |
| 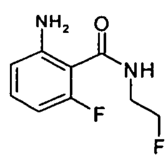 | 201 |
| 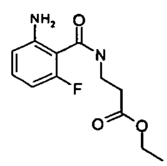 | |
| 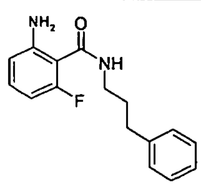 | |

| | |
|---|---|
| 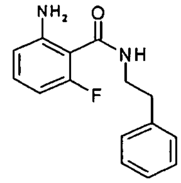 | 245 |
| 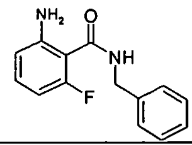 | |

| |
|---|
| 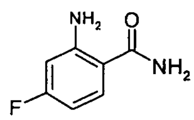 |
| 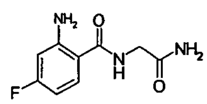 |
| 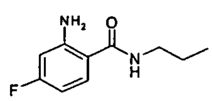 |
| 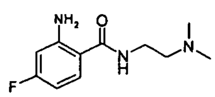 |

**Methode 7**

2-Amino-6-(2-amino-ethoxy)-benzamid

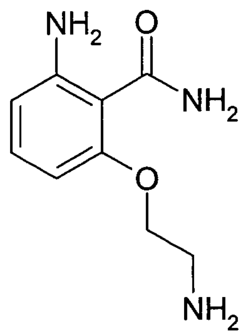

444 µl (7,4 mmol) Ethanolamin werden in 4 ml 1,4-Dioxan gelöst, mit 312 mg (7,8 mmol) Natriumhydrid versetzt und 30 min. bei Raumtemperatur gerührt. Diesem Reaktionsgemisch werden 1,0 g (7,4 mmol) 2-Amino-6-fluorbenzonitril zudosiert und 6 d bei Raumtemperatur gerührt. Anschließend wird das Lösungsmittel im Vakuum entfernt. Das Rohprodukt wird durch Säulenchromatographie gereinigt. Als Trägermaterial dient Kieselgel und als Eluent wird Dichlormethan, dem 10% einer Mischung aus 90% Methanol und 10% gesättigter wässriger Ammoniak-Lösung zugefügt sind, verwendet. 434 mg (2,5 mmol) des gereinigten Zwischenproduktes werden in 5 ml einer 20%-igen Kaliumhydroxid-Lösung in Ethanol gelöst und 2 d bei 90 °C gerührt. Anschließend wird das Lösungsmittel im Vakuum entfernt.
Das Rohprodukt wird durch Säulenchromatographie gereinigt. Als Trägermaterial dient Kieselgel und als Eluent wird Dichlormethan, dem 20% einer Mischung aus 90% Methanol und 10% gesättigter wässriger Ammoniak-Lösung zugefügt sind, verwendet.

| | |
|---|---|
| Ausbeute | 164 mg (0,841 mmol; 11 %) |
| MS (ESI) | 196 $(M+H)^+$ |

Analog zu dieser Methode werden folgende Verbindungen hergestellt. Dabei wird 2-Amino-6-fluorbenzonitril und ein entsprechendes kommerziell erhältlicher Alkohol verwendet.

| | MS (ESI) $(M+H)^+$: |
|---|---|
| 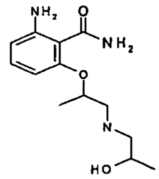 | 268 |
| 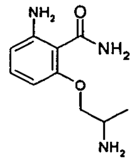 | 210 |
| 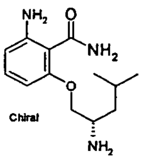 | 252 |
| 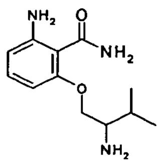 | 238 |
| 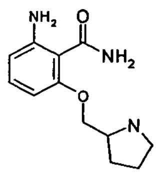 | 236 |
| 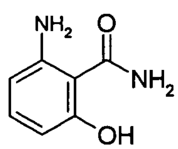 | 153 |
| 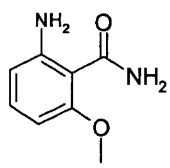 | |

**Methode 8**

2-Amino-benzoesäure-isopropylester

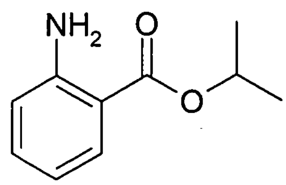

1,0 g (7,3 mmol) Anthranilsäure werden in 5 ml Propan-2-ol gelöst, mit 795 µl (10,9 mmol) Thionylchlorid versetzt und 3 Tage unter Rückfluss erhitzt. Anschließend wird Thionylchlorid im Vakuum entfernt, der Rückstand in 50 ml dest. Wasser aufgenommen und dreimal mit je 30 ml Ethylacetat extrahiert. Die organische Phase wird mit 20 ml einer gesättigten wässrigen $NaHCO_3$-Lsg. gewaschen, mit $MgSO_4$ getrocknet und das Lösungsmittel im Vakuum entfernt.

| | |
|---|---|
| Ausbeute | 156 mg (0,872 mmol; 12 %) |
| MS (ESI) | 180 $(M+H)^+$ |
| $^1$H-NMR | 1.30 (d, 6H), 5.04 - 5.14 (m, 1H), 6.48 - 6.55 (m, 1H), 6.60 (s, 2H), 6.72 - 6.78 (m, 1H), 7.20 - 7.27 (m, 1H), 7.66 - 7.71 (m, 1H) |

Analog zu dieser Methode werden folgende Verbindungen hergestellt. Dabei wird Anthranilsäure und ein entsprechendes kommerziell erhältlicher Alkohol verwendet.

| | **MS (ESI) $(M+H)^+$:** |
|---|---|
| 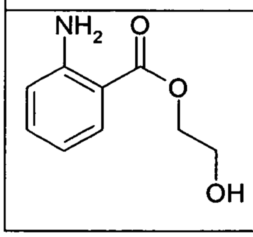 | 182 |

| | |
|---|---|
| 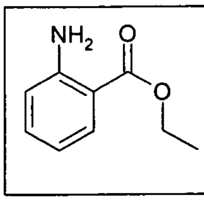 | 166 |

**Methode 9**

1-(2-Amino-phenyl)-propan-1-on

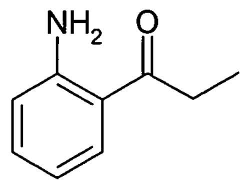

100 mg (0,6 mmol) *N*-(2-Formyl-phenyl)-acetamid (Angew. Chem., Int. Ed. 2002, 41(16), 3028-31) werden in 4 ml Tetrahydrofuran gelöst und auf-78 °C abgekühlt. Anschließend werden bei dieser Temperatur 406 µl (1,2 mmol) einer 3 M Lösung von Ethylmagnesiumbromid in Diethylether zugegeben. Dieses Reaktionsgemisch läßt man über Nacht unter Rühren auf Raumtemperatur kommen. Danach wird das Reaktionsgemisch in 30 ml dest. Wasser eingerührt und dreimal mit je 10 ml Ethylacetat extrahiert. Die organische Phase wird mit $MgSO_4$ getrocknet, das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan:Ethylacetat (1:1) verwendet.
93 mg (0,5 mmol) dieses Zwischenproduktes werden in 6 ml Dichlormethan gelöst, mit 209 mg (2,4 mmol) Mangan (IV)-oxid versetzt und 3 Tage bei Raumtemperatur gerührt. Anschließend wird das überschüssiges Mangan(IV)-oxid über Celite abfiltriert und das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan:Ethylacetat (85:15) verwendet.
44 mg (0,23 mmol) dieses Zwischenproduktes werden in 2 ml Ethanol gelöst, mit 4 ml einer wässrigen 1,0 N Salzsäure versetzt und 2 Tage bei 40 °C gerührt. Anschließend wird das Reaktionsgemisch in 30 ml dest. Wasser eingerührt, mit $Na_2CO_3$ auf pH 7 eingestellt und dreimal mit je 10 ml Ethylacetat extrahiert. Die organische Phase mit $MgSO_4$ getrocknet und das Lösungsmittel im Vakuum entfernt.
Ausbeute: 25 mg (0,168 mmol; 28 %)
MS (ESI): 150 $(M+H)^+$

Analog zu dieser Methode werden folgende Verbindungen hergestellt. Dabei geht man von *N*-(2-Formyl-phenyl)-acetamid und einer entsprechenden kommerziell erhältlichen Grignad-Verbindung aus.

| | MS (ESI) $(M+H)^+$: |
|---|---|
| 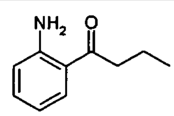 | 164 |
| 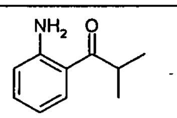 | 164 |
| 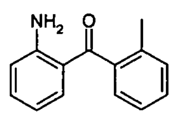 | 212 |

**Methode 10**

4-(2-Acetyl-phenylamino)-2-chlor-5-trifluormethyl-pyrimidin

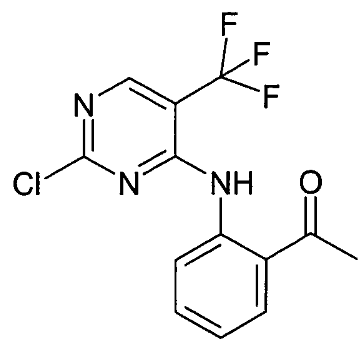

4,23 g (18,5 mmol) 2,4-Dichlor-5-trifluor-methyl-pyrimidin (J. Org. Chem. 1965, 30(3), 835) werden in 2 ml Tetrahydrofuran gelöst, auf - 40 °C abgekühlt und mit 2,20 g (16,0 mmol) 2-Aminoacetophenon und 7,77 ml (44,5 mmol) *N*-Ethyldiisopropylamin versetzt. Nach 3 Tagen bei Raumtemperatur wird das Lösungsmittel im Vakuum entfernt.
Das Rohprodukt wird durch Säulenchromatographie gereinigt. Als Trägermaterial dient Kieselgel und als Eluent wird Dichlormethan verwendet.

| | |
|---|---|
| Ausbeute | 1,38 g (4,4 mmol; 24 %) |
| MS (ESI) | 316/318 $(M+H)^+$Isotopenverteilung $^{35}Cl$/ $^{37}Cl$ |
| $^1H$-NMR | (CDCl3) 2.69 (s, 3H), 7.10 - 7.18 (m, 1H), 7.57 - 7.64 (m, 1H), 7.91 - 7.97 (m, 1H), 8.62 (s, 1H), 8.76 - 8.82 (m, 1H), 12.12 (s, 1H) |

**Methode 11**

4-Amino-3-(2-chlor-phenoxy)-*N*-propyl-benzamid

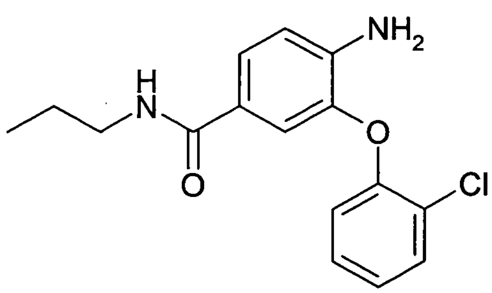

8,4 g (65,3 mmol) Chlorphenol werden in 60 ml N,N-Dimethylformamid gelöst und mit 5,0 g (36,2 mmol) Kaliumcarbonat versetzt. Man erwärmt auf 55°C und tropft 15,0 g (66,3 mmol) 3-Fluoro-4-nitro-*N*-propyl-benzamide, welches in 20 ml N,N-Dimethylformamid gelöst ist, zu und rührt 16 h bei 55°C.
Das Lösungsmittel wird im Vakuum entfernt und der Rückstand mit 100 ml Wasser versetzt. Die entstehenden Kristalle werden abgesaugt und getrocknet. 14 g (41,8 mmol) dieses Zwischenproduktes werden in 150 ml THF gelöst und mit 1 g Raney-Nickel versetzt. Die Reaktionsmischung wird 16 h bei RT und 3 bar WasserstoffDruck gerührt. Anschließend wird vom Katalysator abfiltriert und das Lösungsmittel im Vakuum entfernt. Der Rückstand wird in Butylmethylether aufgenommen und mit 16 ml einer 5 molaren Lösung von HCl (80 mmol) in Isopropanol versetzt. Die Kristalle werden

abgesaugt und getrocknet.

| | |
|---|---|
| Ausbeute | 13,5 g (44,4 mmol; 68 %) |
| MS (ESI) | 305/307 $(M+H)^+$ Isotopenverteilung $^{35}Cl$ / $^{37}Cl$ |

Analog zu dieser Methode werden folgende Verbindungen hergestellt.

| | MS (ESI) $(M+H)^+$: |
|---|---|
| 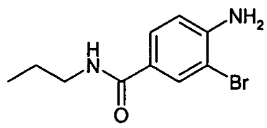 | 257/259 |
| 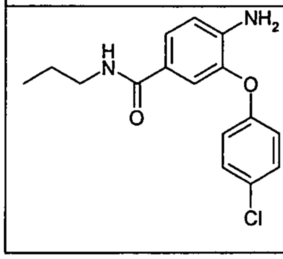 | |

| |
|---|
| 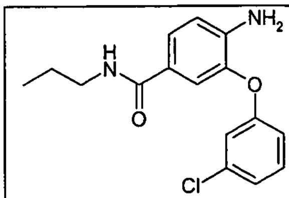 |
| 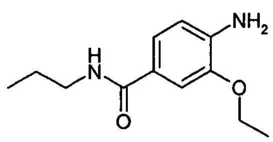 |
| 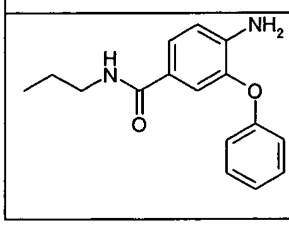 |

**Methode 12**

2-(4-Carboxyamino-2-methoxy-phenylamino)-4-chlor-5-trifluormethyl-pyrimidin

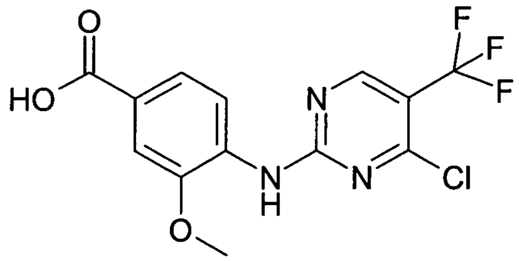

7,36 g (44,0 mmol) 4-Amino-3-methoxybenzoesäure werden in 80 ml einer wässrigen Phosphatpuffer-Lösung (pH 6,3) suspendiert und mit 9,5 g (44,0 mmol) 2,4-Dichlor-5-trifluor-methyl-pyrimidin, welches in 240 ml 1,4-Dioxan gelöst ist, versetzt. Nach 4 h bei 100 °C wird das Reaktionsgemisch bei 0°C zur Auskristallisation gebracht. Der Niederschlag wird abfiltriert, das Filtrat wird mit 150 ml Ethylacetat versetzt und zweimal mit je 200 ml einer gesättigten wässrigen Natriumhydrogencarbonat-Lösung gewaschen. Die organische Phase wird mit $MgSO_4$ getrocknet und das Lösungsmittel im Vakuum entfernt. Das Rohprodukt wird in 10 ml n-Hexan suspendiert und unter Rückfluss erhitzt.

Der Niederschlag wird abfiltriert, in 48 ml einer gesättigten wässrigen

Natriumhydrogencarbonat-Lösung suspendiert und 1 h bei 65 °C erhitzt. Anschließend wird die Lösung bei 0°C zur Auskristallisation gebracht. Der Niederschlag wird abfiltriert, das Filtrat wird mit 1 N wässriger Salzsäure angesäuert und mit 100 ml Essigester versetzt. Die organische Phase wird abgetrennt, mit Magnesiumsulfat getrocknet und das Lösungsmittel im Vakuum entfernt.
Der Rückstand wird in Ethylacetat umkritallisiert.

| | |
|---|---|
| Ausbeute | 330 mg (0,95 mmol, 2 %) |
| MS (ESI) | 348 $(M+H)^+$ |
| $^1$H-NMR | 1.55 (s, 1H), 4.01 (s, 3H), 7.61 - 7.64 (m, 1H), 7:79 - 7.85 (m, 1H), 8.34 (s, 1H), 8.59 - 8.63 (m, 1H), 8.66 (s, 1H) |

**Methode 13**

4-(4-Amino-cyclohexyl)-morpholin

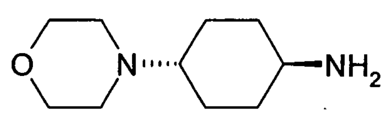

Dibenzyl-4-morpholino-cyclohexylamin

3,9 g (30 mmol)) 4-Dibenzylcyclohexanon werden in 100 mL Dichlormethan gelöst und mit 3,9 g (45 mmol) Morpholin und 9,5 g (45 mmol) Natriumtriacetoxyborhydrid 12 h bei RT gerührt. Anschließend wird mit Wasser und Kaliumcarbonat versetzt, die organische Phase abgetrennt, getrocknet und im Vakuum das Lösungsmittel abgezogen. Das Rohprodukt wird durch Säulenchromatographie gereinigt. Als Trägermaterial dient Kieselgel und als Eluent wird

Essigester, dem 10% einer Mischung aus 90% Methanol und 10% gesättigter wässriger Ammoniak-Lösung zugefügt sind, verwendet. Die geeigneten Fraktionen wurden im Vakuum eingeengt.

| | |
|---|---|
| Ausbeute | 6,6 g (18 mmol, 60%) cis-Isomer<br>2 g (5,4 mmol, 18%) trans-Isomer. |

trans-4-Morpholino-cyclohexylamin

7,2 g (16,4 mmol) trans-Dibenzyl-4-morpholino-cyclohexylamin wurden in 100 mL MeOH gelöst und an 1,4 g Pd/C (10%) bei 30-50 °C hydriert. Das Lösungsmittel wurde im Vakuum abgezogen und der Rückstand aus Ethanol und konz. HCl kristallisiert.

| | |
|---|---|
| Ausbeute | 3,9 g (15,2 mmol, 93%) |
| Schmelzpunkt | 312°C |

**Methode 14**

(2-Amino-6-fluor-phenyl)-*o*-tolyl-methanon

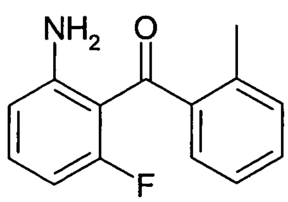

2-Amino-6-fluor-benzoesäuremethylester

5,00 g (31,6 mmol) 2-Amino-6-fluorbenzoesäure werden in 50 ml Methanol gelöst und mit 7,9 ml (31,6 mmol) einer 4 M Lösung von HCL in 1,4-Dioxan versetzt und 10 min. bei Raumtemperatur gerührt. Das Reaktionsgemisch wird tropfenweise mit 3,45 ml (47,4 mmol) Thionylchlorid versetzt und unter Rückfluss 3 h erhitzt. Anschließend wird das Reaktionsgemisch mit 150 ml einer gesättigten, wässrigen $NaHCO_3$-Lösung neutralisiert und viermal mit je 100 ml Ethylacetat extrahiert. Die organische Phase wird mit $MgSO_4$ getrocknet und das Lösungsmittel im Vakuum entfernt.

| | |
|---|---|
| Ausbeute | 1,57 g (9,3 mmol, 29 %) |

(2-Amino-6-fluor-phenyl)-methanol

676,3 mg (17,8 mmol) Lithiumaluminiumhydrid werden unter $N_2$-Atmospäre in 65 ml Diethylether vorgelegt und auf 0 °C abgekühlt. Dazu werden langsam 2,01 g (11,9 mmol) 2-Amino-6-fluor-benzoesäuremethylester, gelöst in 65 ml Diethylether, getropft und 2 h bei 0 °C gerührt. Anschließend wird das Reaktionsgemisch bei 0 °C tropfenweise mit 100 ml dest. $H_2O$ versetzt. Die wässrige Phase wird zweimal mit je 100 ml Diethylether extrahiert. Die vereinigten organischen Phasen werden einmal mit 100 ml einer gesättigten, wässrigen NaCl-Lösung gewaschen, mit $MgSO_4$ getrocknet und das Lösungsmittel im Vakuum entfernt.

| | |
|---|---|
| Ausbeute | 1,44 g (10,2 mmol, 86 %) |

2-Amino-6-fluor-benzaldehyd

1,44 g (10,2 mmol) (2-Amino-6-fluor-phenyl)-methanol werden in 120 ml Chloroform gelöst, mit 4,43 g (51,0 mmol) Mangan(IV)-oxid versetzt und 2 d bei Raumtemperatur gerührt. Anschließend wird überschüssiges Mangan(IV)-oxid über Celite abfiltriert und das Lösungsmittel im Vakuum entfernt.

| | |
|---|---|
| Ausbeute | 1,36 g (9,78 mmol, 96 %) |

*N*-(3-Fluor-2-formyl-phenyl)-acetamid

1,37 g (9,85 mmol) 2-Amino-6-fluor-benzaldehyd werden mit 20 ml Essigsäureanhydrid versetzt und 4 h bei 70 °C erhitzt. Anschließend wird das Reaktionsgemisch in 200 ml dest. Wasser eingerührt, mit $Na_2CO_3$ auf pH 7 eingestellt und dreimal mit je 50 ml Ethylacetat extrahiert. Danach wird die organische Phase mit $MgSO_4$ getrocknet, das Lösungsmittel im Vakuum entfernt.

Das Rohprodukt wird durch Säulenchromatographie gereinigt. Als Trägermaterial dient Kieselgel und als Eluent wird ein Gemisch bestehend aus Cyclohexan : Ethylacetat (80:20) verwendet.

| | |
|---|---|
| Ausbeute | 1,40 g (7,73 mmol, 79 %) |
| $^1$H-NMR | 2.17 (s, 3H), 7.04 - 7.12 (m, 1H), 7.66 - 7.74 (m, 1H), 8.13 - 8.18 (m, 1H), 10.22 (s, 1H), 11.00 (s, 1H) |

*N*-[3-Fluor-2-(hydroxyl-*o*-tolyl-methyl)-phenyl]-acetamid

100 mg (0,83 mmol) *N*-(3-Fluor-2-formyl-phenyl)-acetamid werden in 4 ml Tetrahydrofuran gelöst und auf -78 °C abgekühlt. Anschließend werden bei dieser Temperatur 1,66 ml (3,3 mmol) einer 2 M Lösung von *o*-Tolylmagnesiumbromid in Diethylether zugegeben. Dieses Reaktionsgemisch wird über Nacht unter Rühren auf Raumtemperatur aufgetaut. Danach wird das Reaktionsgemisch in 30 ml dest. Wasser eingerührt und dreimal mit je 10 ml Ethylacetat extrahiert. Die organische Phase wird mit $MgSO_4$ getrocknet, das Lösungsmittel im Vakuum entfernt.
Das Rohprodukt wird durch Säulenchromatographie gereinigt. Als Trägermaterial dient Kieselgel und als Eluent wird ein Gemisch bestehend aus Cyclohexan : Ethylacetat (80:20) verwendet.

| | |
|---|---|
| Ausbeute | 205 mg (0,75 mmol, 90 %) |

*N*-[3-Fluor-2-(2-methyl-benzoyl)-phenyl]-acetamid

205 mg (0,75 mmol) *N*-[3-Fluor-2-(hydroxyl-*o*-tolyl-methyl)-phenyl]-acetamid werden in 9 ml Chloroform gelöst, mit 652 mg (7,5 mmol) Mangan(IV)-oxid versetzt und 3 d bei Raumtemperatur gerührt. Anschließend wird überschüssiges Mangan(IV)-oxid über Celite abfiltriert, das Lösungsmittel im Vakuum entfernt.
Das Rohprodukt wird durch Säulenchromatographie gereinigt. Als Trägermaterial dient Kieselgel und als Eluent wird ein Gemisch bestehend aus Cyclohexan : Ethylacetat (80:20) verwendet.

| | |
|---|---|
| Ausbeute | 142 mg (0,52 mmol, 70 %) |

(2-Amino-6-fluor-phenyl)-*o*-tolyl-methanon

142 mg (0,52 mmol) *N*-[3-Fluor-2-(2-methyl-benzoyl)-phenyl]-acetamid werden in 2 ml Ethanol gelöst, mit 2 ml konz. Salzsäure versetzt und 4 h bei 70 °C gerührt. Anschließend wird das Reaktionsgemisch in 30 ml dest. Wasser eingerührt, mit Natriumcarbonat auf pH 7 eingestellt und dreimal mit je 10 ml Ethylacetat extrahiert. Danach wird die organische Phase mit $MgSO_4$ getrocknet und das Lösungsmittel im Vakuum entfernt.

| | |
|---|---|
| Ausbeute | 1,40 g (7,73 mmol, 79 %) |
| MS (ESI) | 230 $(M+H)^+$ |
| $^1$H-NMR | 2.27 (s, 3H), 6.18 - 6.26 (m, 1H), 6.64 - 6.69 (m, 1H), 6.93 (s, 2H), 7.17 - 7.31 (m, 4H), 7.32 - 7.40 (m, 1H) |

Analog zu dieser Methode werden folgende Verbindungen hergestellt.

| | MS (ESI) $(M+H)^+$: | NMR: |
|---|---|---|
| 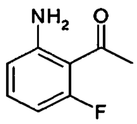 | 154 | 6.26 – 6.35 (m, 1H), 6.55 – 6.61 (m, 1H), 7.15 - 7.23 (m, 1H), 7.28 (s, 2H) |
| 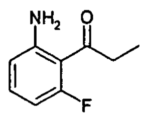 | 168 | 1.05 (t, 3H), 2.82 - 2.90 (m, 2H), 6.27 – 6.34 (m, 1H), 6.55 – 6.61 (m, 1H), 7.12 - 7.23 (m, 3H) |
| 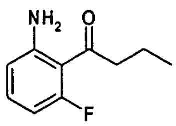 | 182 | 0.90 (t, 3H), 1.55 - 1.65 (m, 2H), 2.78 - 2.85 (m, 2H), 6.27 – 6.34 (m, 1H), 6.55 – 6.61 (m, 1H), 7.13 (s, 2H), 7.14 - 7.21 (m, 1H) |

**Methode 15**

(2-Amino-phenyl)-[2-(piperidin-yl-1-carbonyl)-phenyl]-methanon

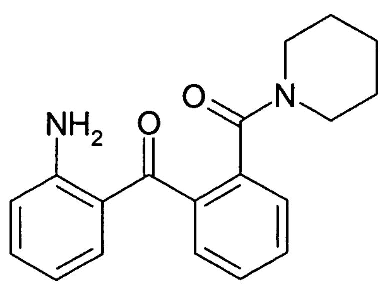

200 mg (0,81 mmol) 2-Aminobenzophenon-2-carbonsäure, 81 µl (0,81 mmol) Piperidin, 425 µl (2,43 mmol) *N*-Ethyldiisopropylamin werden in 1 ml Tetrahydrofuran gelöst. Diesem Reaktionsgemisch werden 265 mg (0,81 mmol) TBTU zugesetzt und über Nacht bei Raumtemperatur gerührt. Anschließend wird das Reaktionsgemisch in 20 ml dest. Wasser eingerührt und dreimal mit je 5 ml Ethylacetat extrahiert. Danach wird die organische Phase über Alox B filtriert, mit $MgSO_4$ getrocknet, das Lösungsmittel im Vakuum entfernt.
Das Rohprodukt wird durch Säulenchromatographie gereinigt. Als Trägermaterial dient Kieselgel und als Eluent wird Dichlormethan, dem 2% einer Mischung aus 90% Methanol und 10% gesättigter wässriger Ammoniak-Lösung zugefügt sind, verwendet.

| | |
|---|---|
| Ausbeute | 89 mg (0,29 mmol, 36 %) |
| MS (ESI) | 309 $(M+H)^+$ |

(fortgesetzt)

| | |
|---|---|
| $^1$H-NMR | 1.40 - 1.52 (m, 4H), 1.53 - 1.61 (m, 2H), 3.15 - 3.27 (m, 2H), 3.35 - 3.45 (m, 2H), 6.42 - 6.48 (m, 1H), 6.79 - 6.83 (m, 1H), 7.10 - 7.14 (m, 1H), 7.15 (s, 2H), 7.22 - 7.29 (m, 1H), 7.35 - 7.42 (m, 2H), 7.46 - 7.52 (m, 1H), 7.52 -7.58 (m, 1H) |

**Biologische Eigenschaften**

Wie durch DNA-Färbung mit darauf folgender FACS Analyse gezeigt werden konnte, ist die, durch die erfindungsgemäßen Verbindungen bewirkte, Proliferationsinhibition vor allem durch einen Arrest der Zellen in der G2/M Phase des Zellzyklus vermittelt. Die Zellen arretieren abhängig von dem verwendeten Zelltyp für eine bestimmte Zeitspanne in dieser Zellzyklus Phase, bevor der programmierte Zelltod eingeleitet wird. Ein Arrest in der G2/M Phase des Zellzyklus kann z.B. durch die Inhibition spezifischer Zellzykluskinasen ausgelöst werden. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphome und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001). Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Zytostatika, Steroide oder Antikörper, verwendet werden.

**Beispiel PLK-1 Kinaseassay**

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10$^7$ Sf21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10$^8$ Sf21 Zellen in insgesamt 180 ml Medium in eine neue Spinnerflasche ausgesät. Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 μM) und die Suspension weiter inkubiert. Die Zellzahl wird bestimmt, die Zellen abzentrifugiert (5 Minuten, 4°C, 800 rpm) und 1x mit PBS (8 g NaCl/l, 0,2 g KCl/l, 1,44 g $Na_2HPO_4$/l, 0,24 g $KH_2PO4$/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM $MgCl_2$, 1 mM DTT, 5 μg/ml Leupeptin, 5 μg/ml Aprotinin, 100 μM NaF, 100 μM PMSF, 10 mM β-Glycerolphosphat, 0.1 mM $Na_3VO_4$, 30 mM 4-Nitrophenylphosphate) zu 1x10$^8$ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCl pH=8,0, 120 mM NaCl, 20 mM reduziertes Glutathion (Sigma G-4251), 10 mM $MgCl_2$, 1 mM DTT) von den Beads eluiert. Die Proteinkonzentration wird mittels Bradford Assay bestimmt.

**Assay**

In einem Napf einer 96-Loch Rundbodenplatte (Fa. Greiner bio-one, PS-Microtiterplatte Nr.650101) werden folgende Komponenten zusammengefügt:

- 10 μl zu testende Verbindung in variabler Konzentration (z.B. beginnend bei 300 μM, und Verdünnung in 1:3) in 6% DMSO, 0,5 mg/ml Casein (Sigma C-5890), 60 mM β-Glycerophosphat, 25 mM MOPS pH=7,0, 5 mM EGTA, 15 mM $MgCl_2$, 1 mM DTT
- 20 μl Substratlösung (25 mM MOPS pH=7,0, 15 mM $MgCl_2$, 1 mM DTT, 2,5 mM EGTA, 30 mM β-Glycerophosphat, 0,25 mg/ml Casein)

- 20 µl Enzymverdünnung (1:100 Verdünnung des Enzymstocks in 25 mM MOPS pH=7,0, 15 mM $MgCl_2$, 1 mM DTT)
- 10 µl ATP Lösung (45 µM ATP mit 1,11x$10^6$ Bq/ml gamma-P33-ATP). Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35µl Szintillationslösung (Ready-Safe; Beckmann) pro Napf wird die Platte mit Sealingtape zugeklebt und die präzipitierte Menge P33 mit dem Wallac Betacounter gemessen. Die Messdaten werden mit der Standard Graphpad Software (Levenburg-Marquard Algorhythmus) ausgewertet.

Die Wirkung der erfindungsgemäßen Verbindungen wird im Cytotoxizitätstest an kultivierten humanen Tumorzellen und/oder in einer FACS-Analyse, beispielsweise an HeLa S3-Zellen, bestimmt. Die Verbindungen zeigen in beiden Testmethoden eine gute bis sehr gute Wirksamkeit, d.h. beispielsweise einen EC50-Wert im HeLa S3-Cytotoxizitätstest kleiner 5 µmol/L, in der Regel kleiner 1 µmol/L.

**Messung der Cytotoxizität an kultivierten humanen Tumorzellen**

Zur Messung der Cytotoxizität an kultivierten humanen Tumorzellen werden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend werden die HeLa S3 Zellen in 96-well Platten (Costar) mit einer Dichte von 1000 Zellen pro well eingebracht und über Nacht in einem Inkubator (bei 37°C und 5 % CO2) inkubiert, wobei auf jeder Platte 6 wells nur mit Medium gefüllt werden (3 wells zur Mediumkontrolle, 3 wells zur Inkubation mit reduzierten AlamarBlue Reagenz). Die Wirksubstanzen werden in verschiedenen Konzentrationen (gelöst in DMSO; DMSO-Endkonzentration: 0.1%) zu den Zellen zugegeben (jeweils als Dreifachbestimmung). Nach 72 Stunden Inkubation werden zu jedem well 20 µl AlamarBlue Reagenz (AccuMed International) zugesetzt, und die Zellen für weitere 5-7 Stunden inkubiert. Zur Kontrolle wird zu 3 wells je 20 µl reduziertes AlamarBlue Reagenz gegeben (AlamarBlue Reagenz, das für 30 min autoklaviert wird). Nach Inkubation wird der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wird in Prozent der Kontrolle (HeLa S3 Zellen ohne Inhibitor) berechnet und die Wirkstoffkonzentration, die die Zellaktivität zu 50% hemmt (IC50) abgeleitet. Die Werte werden hierbei aus dem Mittelwert von drei Einzelbestimmungen - unter Korrektur des Leerwertes (Mediumkontrolle)- berechnet.

**FACS- Analyse**

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.
Für eine PI-Färbung werden beispielsweise 1x$10^6$ HeLa S3 Zellen auf eine 75 cm2 Zellkulturflasche ausgesät, nach 24 h wird entweder 0.1 % DMSO als Kontrolle zugesetzt, bzw. die Substanz in verschiedenen Konzentrationen (in 0.1% DMSO). Die Zellen werden für 24 h mit der Substanz bzw. mit DMSO inkubiert, bevor die Zellen 2 x mit PBS gewaschen und dann mit Trypsin /EDTA abgelöst werden. Die Zellen werden zentrifugiert (1000 Upm, 5 min, 4°C), und das Zellpellet 2 x mit PBS gewaschen, bevor die Zellen in 0.1 ml PBS resuspendiert werden. Anschließend werden die Zellen für 16 Stunden bei 4°C oder alternativ für 2 Stunden bei -20°C mit 80% Ethanol fixiert. Die fixierten Zellen werden zentrifugiert (1000 Upm, 5min, 4°C), mit PBS gewaschen und anschließend nochmals zentrifugiert. Das Zellpellet wird in 2 ml 0.25% Triton X-100 in PBS resuspendiert, und 5 min auf Eis inkubiert, bevor 5 ml PBS zugeben werden und erneut zentrifugiert wird. Das Zellpellet wird in 350 µl PI Färbelösung (0.1 mg/ml RNase A (Sigma, No. R-4875), 10 µg/ml Prodium Iodid (Sigma, No. P-4864) in 1 x PBS) resuspendiert. Die Zellen werden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einem Argonlaser (500 mW, Emission 488 nm), und dem DNA Cell Quest Programm (BD). Die logarithmische PI Fluoreszenz wird mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend werden die erfindungsgemäßen Verbindungen auch auf weiteren Tumorzellen getestet. Beispielsweise sind diese Verbindungen auf Karzinomen verschiedenster Gewebe (z. Bsp. Brust (MCF7); Colon (HCT116), Kopf-Hals (FaDu), Leber (HepG2), Lunge (NCI-H460), Magen (NCI-N87), Pankreas (BxPC-3), Prostata

(DU145)), Sarkome (z. Bsp. SK-UT-1B, Saos-2), Leukämien und Lymphome (z. Bsp. HL-60, THP-1, Raji, Jurkat, GRANTA-519) und anderen Tumoren (z. Bsp. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfmdungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzukker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer

Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

Pharmazeutische Formulierungsbeispiele

| A) Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) Tabletten | pro Tablette |
|---|---|
| Wirkstoff | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) Ampullenlösung | |
|---|---|
| Wirkstoff | 50 mg |
| Natriumchlorid | 50 mg |
| Aqua pro inj . | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

**Patentansprüche**

**1.** Verbindungen der allgemeinen Formel (1)

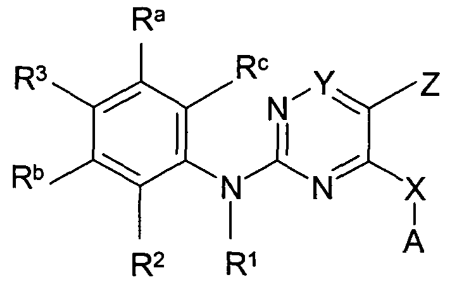

**(1)**

worin

**X** $-NR^{1a}$, O oder S, und

**Y** CH oder N, und

**Z** Wasserstoff, Halogen, $C_{1-3}$-Alkyl, $C_{2-3}$-Alkenyl, $C_{2-3}$-Alkinyl, Halogen-$C_{1-3}$-Alkyl-, -COH, -C(=O)-$C_{1-3}$-Alkyl, -C(=O)-$C_{2-3}$-Alkenyl, -C(=O)-$C_{2-3}$-Alkinyl, -C(=O)$C_{1-3}$-Alkyl-Halogen und Pseudohalogen; und

**A** ausgewählt ist aus den Formeln (i) oder (ii)

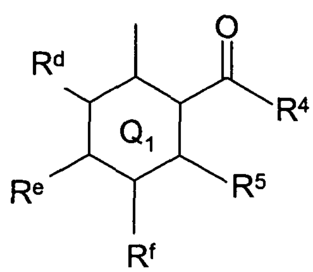

(i)

oder

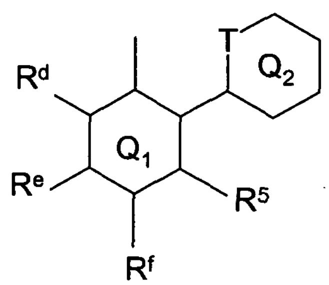

(ii)

und

**$Q_1$** mono- oder bizyklische Arylverbindungen; und

**$Q_2$** mono- oder bizyklische Heteroarylverbindungen; und

**T** N, O oder S, und

**$R^1$ und $R^{1a}$** Wasserstoff oder Methyl, und

**$R^2$** ein Rest ausgewählt aus der Gruppe bestehend aus -Cl, -Br, -I, $-OR^6$, $-C(=O)R^6$, $-C(=O)NR^6R^7$, $-NR^6R^7$, $-NR^6C(=O)R^7$, $-NR^6SO_2R^7$, $-N=CR^6R^7$, $-SR^6$, $-SOR^6$, $-SO_2R^6$, $-SO_2NR^6R^7$ und Pseudohalogen, oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl , Heterocyclyl und Heteroaryl, wobei der/die Substituenten gleich oder verschieden sein können und ausgewählt werden aus der Gruppe bestehend aus Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6NR^7$, $-NR^6SO_2NR^7R^8$, $-OSO_2NR^7R^8$ und Pseudohalogen; und

**$R^a$, $R^b$, $R^c$, $R^d$, $R^e$ und $R^f$** jeweils unabhängig voneinander ein Rest ausgewählt aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N{=}CR^6R^7$, $-SR^6$, $-SOR^6$, $-SO_2R^6$, $-SO_2NR^6NR^7$, $-NR^6SO_2NR^7R^8$, $-OSO_2NR^7R^8$ und Pseudohalogen; oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N{=}CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^3$** ausgewählt aus den Formeln (iii) - (ix),

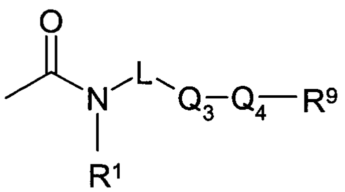

(iii)

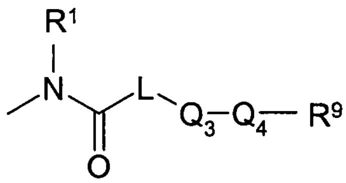

(iv)

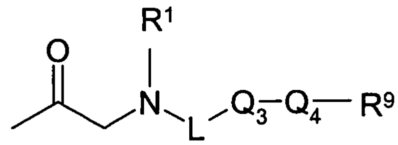

(v)

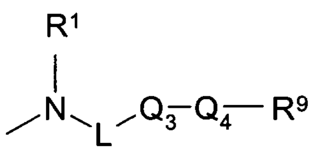

(vi)

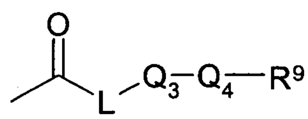

(vii)

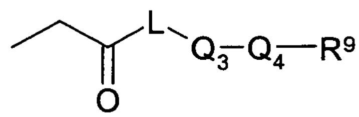

(viii)

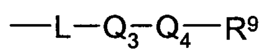

(ix)

und

**$R^4$** ein Rest ausgewählt aus der Gruppe bestehend aus Wasserstoff, $-OR^6$, $-C(=O)R^6$, $-C(=O)OR^6$, $-C(=O)NR^6R^7$, $-NR^6R^7$, $-NR^6C(=O)R^7$ $-NR^6C(=O)OR^7$, $-NR^6C(=O)NR^7R^8$, $-NR^6SO_2R^7$, $-N{=}CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen,
oder ein Rest ausgewählt aus der Gruppe bestehend aus gegebenenfalls einfach oder mehrfach substituiertem $C_{1-8}$-Alkyl, $C_{2-10}$-Alkenyl, $C_{2-10}$-Alkinyl, $C_{3-8}$-Cycloalkyl, Aryl, Heterocyclyl und Heteroaryl, wobei der/die Substituent(en) gleich oder verschieden sein können und ausgewählt werden aus der Gruppe bestehend aus $C_{1-8}$-Alkyl, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N{=}CR^6R^7$, $-SR^6$, $-SOR^6$, $-SO_2R^6$, $-SO_2NR^6R^7$, $-NR^6SO_2NR^7R^8$,

$-OSO_2NR^7R^8$ und Pseudohalogen; und

**$R^5$** Wasserstoff, Halogen, $-CF_3$, $C_{1-3}$-Alkyl oder $-OR^6$; und

**$R^6$, $R^7$ und $R^8$** jeweils unabhängig voneinander Wasserstoff oder ein Rest ausgewählt aus der Gruppe bestehend aus gegebenenfalls einfach oder mehrfach substituiertem $C_{1-5}$-Alkyl, $C_{2-5}$-Alkenyl, $C_{2-5}$-Alkinyl, $C_{3-10}$-Cycloalkyl, Aryl, Heterocyclyl und Heteroaryl, wobei der/die Substituenten gleich oder verschieden sein können und ausgewählt werden aus der Gruppe bestehend aus $C_{3-10}$-Cycloalkyl, Aryl, Heterocyclyl, Heteroaryl, Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}C(=O)R^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$und Pseudohalogen; und

**L** eine Bindung oder ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$Q_3$ und $Q_4$** unabhängig voneinander eine Bindung oder ein Rest ausgewählt aus der Gruppe bestehend aus gegebenenfalls einfach oder mehrfach substitutierten monooder bizyklische Heterocyclyl wobei der/die Substituent(en) gleich oder verschieden sein können und ausgewählt werden aus der Gruppe bestehend aus Methyl, Ethyl, Halogen, $-NH_2$, -OH und Pseudohalogen; und

**$R^9$** ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen-, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)NR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$R^{10}$, $R^{11}$ und $R^{12}$** jeweils unabhängig voneinander Wasserstoff oder ein Rest ausgewählt aus der Gruppe bestehend aus gegebenenfalls einfach oder mehrfach substituiertem $C_{1-8}$-Alkyl, $C_{2-8}$-Alkenyl, $C_{2-8}$-Alkinyl, $C_{3-10}$-Cycloalkyl, Aryl, Heterocyclyl und Heteroaryl, wobei der/die Substituent (en) gleich oder verschieden sein können und ausgewählt werden aus der Gruppe bestehend aus Halogen, $-NH_2$, -OH und Pseudohalogen;

gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, bedeuten.

**2.** Verbindungen nach Anspruch 1, wobei

**X** $-NR^{1a}$ oder Sauerstoff, und

**A** ausgewählt ist aus den Formeln (i) oder (ii)

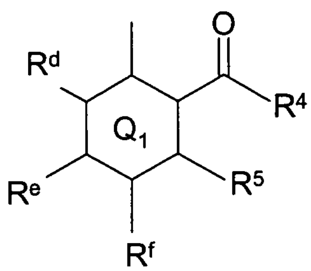

(i)

oder

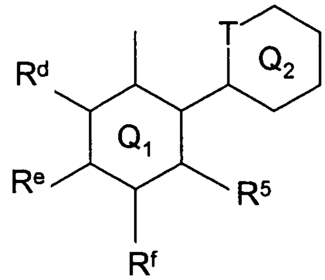

(ii)

und

**$Q_1$** mono- oder bizyklische Arylverbindungen; und

**$Q_2$** monozyklische Heteroarylverbindungen; und

**T** N, O oder S, und

**$R^1$** und **$R^{1a}$** Wasserstoff; und

**$R^3$** Formel (iii),

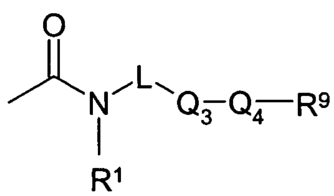

(iii)

bedeuten und die übrigen Reste wie vorstehend erwähnt definiert sind.

3. Verbindungen nach Anspruch 1 oder 2, wobei

**Y** CH; und

**$Q_1$** monozyklische Arylverbindungen

bedeuten und die übrigen Reste wie vorstehend erwähnt definiert sind.

4. Verbindungen nach Anspruch 1 - 3, wobei

**$R^c$** ein Rest ausgewählt aus der Gruppe bestehend aus Wasserstoff, -F, -Cl, Methyl und Ethyl

bedeutet und die übrigen Reste wie vorstehend erwähnt definiert sind.

5. Verbindungen nach Anspruch 1-4, wobei

**$R^a$** und **$R^b$** jeweils unabhängig voneinander Wasserstoff oder ein gegebenenfalls einfach oder mehrfach substituierter Rest ausgewählt aus der Gruppe bestehend aus $C_{1-2}$-Alkyl, $C_2$-Alkenyl, $C_2$-Alkinyl, $C_{3-6}$-Cycloalkyl, Aryl, Heterocyclyl und Heteroaryl, wobei der/die Substituenten gleich oder verschieden sein

können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6$, $-SO_2NR^7R^8$, $-OSO_2NR^7R^8$ und Pseudohalogen

bedeuten und die übrigen Reste wie vorstehend erwähnt definiert sind.

**6.** Verbindungen nach Anspruch 1-5, wobei

**$R^a$** und **$R^b$** Wasserstoff

bedeuten und die übrigen Reste wie vorstehend erwähnt definiert sind.

**7.** Verbindungen nach Anspruch 1- 6, wobei

**Z** Halogen-$C_{1-3}$-Alkyl-, -COH, -C(=O)-$C_{1-3}$-Alkyl, -C(=O)-$C_{2-3}$-Alkenyl, -C(=O)-$C_{2-3}$-Alkinyl, -C(=O)$C_{1-3}$-Alkyl-Halogen und Pseudohalogen

bedeutet und die übrigen Reste wie vorstehend erwähnt definiert sind.

**8.** Verbindungen nach Anspruch 1 - 7, wobei

**Z** $C_{1-3}$-Fluoralkyl- und
**Y** CH

bedeuten und die übrigen Reste wie vorsehend erwähnt definiert sind.

**9.** Verbindungen - oder deren pharmazeutisch wirksamen Salze - nach Ansprüchen 1 bis 8 zur Verwendung als Arzneimittel.

**10.** Verbindungen - oder deren pharmazeutisch wirksamen Salze - nach Ansprüchen 1 bis 8 zur Herstellung eines Arzneimittels mit antiproliferativer Wirkung.

**11.** Verbindungen - oder deren pharmazeutisch wirksamen Salze - nach Ansprüchen 1 bis 8 zur Herstellung eines Arzneimittels mit antiproliferativer Wirkung mit einem selektiven, kinaseinhibierendem Wirkmechanismus.

**12.** Verbindungen - oder deren pharmazeutisch wirksamen Salze - nach Ansprüchen 1 bis 8 zur Herstellung eines Arzneimittels mit antiproliferativer Wirkung mit einem PLK inhibierendem Wirkmechanismus.

**13.** Pharmazeutische Zubereitungen, enthaltend als Wirkstoff eine oder mehrere Verbindungen der allgemeinen Formel (I) gemäß einem der Ansprüche 1 bis 8 oder deren physiologisch verträgliche Salze gegebenenfalls in Kombination mit üblichen Hilfs-und/oder Trägerstoffen.

**14.** Verwendung einer Verbindung nach Anspruch 1 bis 8 zur Herstellung eines Arzneimittels zur Behandlung und/oder Prävention von Krebs, Infektionen, Entzündungs-und Autoimmunerkrankungen.

**15.** Pharmazeutische Präperation umfassend eine Verbindung der allgemeinen Formel (1)

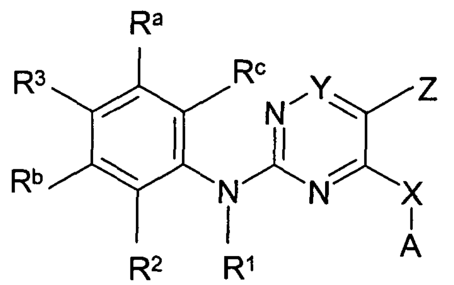

**(1)**

worin

**X** $-NR^{1a}$, O oder S, und

**Y** CH oder N, und

**Z** Wasserstoff, Halogen, $C_{1-3}$-Alkyl, $C_{2-3}$-Alkenyl, $C_{2-3}$-Alkinyl, Halogen-$C_{1-3}$-Alkyl-, -COH, -C(=O)-$C_{1-3}$-Akyl, -C(=O)-$C_{2-3}$-Alkenyl, -C(=O)-$C_{2-3}$-Alkinyl, -C(=O)$C_{1-3}$-Alkyl-Halogen und Pseudohalogen; und

**A** ausgewählt ist aus den Formeln (i) oder (ii)

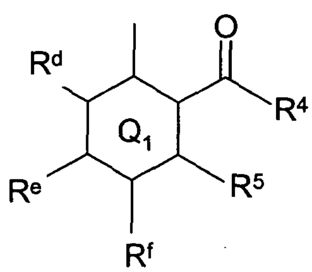

(i)

oder

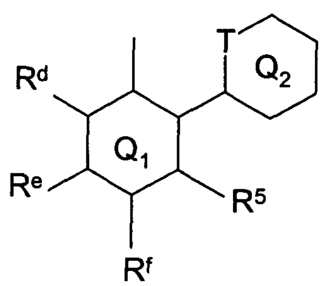

(ii)

und

**$Q_1$** mono- oder bizyklische Arylverbindungen; und

**$Q_2$** mono- oder bizyklische Heteroarylverbindungen; und

**T** N, O oder S, und

**$R^1$** und **$R^{1a}$** Wasserstoff oder Methyl, und

**$R^2$** ein Rest ausgewählt aus der Gruppe bestehend aus -Cl, -Br, -I, $-OR^6$, $-C(=O)R^6$, $-C(=O)NR^6R^7$, $-NR^6R^7$, $-NR^6C(=O)R^7$, $-NR^6SO_2R^7$, $-N=CR^6R^7$, $-SR^6$, $-SOR^6$, $-SO_2R^6$, $-SO_2NR^6R^7$ und Pseudohalogen, oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl , Heterocyclyl und Heteroaryl, wobei der/die Substituenten gleich oder verschieden sein können und ausgewählt werden aus der Gruppe bestehend aus Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und

Pseudohalogen; und

**$R^a$, $R^b$, $R^c$, $R^d$, $R^e$** und **$R^f$** jeweils unabhängig voneinander ein Rest ausgewählt aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; oder ein gegebenenfalls einfach oder mehrfach substituierter Rest ausgewählt aus der Gruppe bestehend aus $C_{1-6}$-Alkyl, $C_{2-6}$-Alkenyl, $C_{2-6}$-Alkinyl, $C_{3-6}$-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, $-NO_2$, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^3$** ausgewählt aus den Formeln (iii) - (ix),

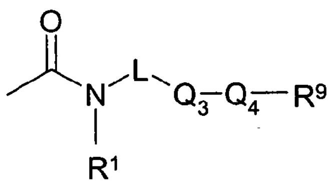

(iii)

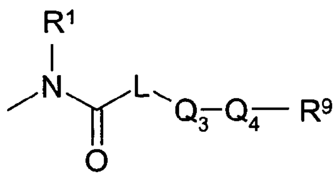

(iv)

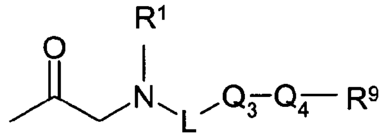

(v)

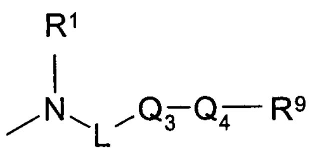

(vi)

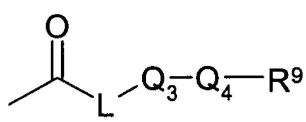

(vii)

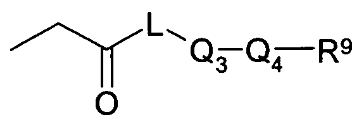

(viii)

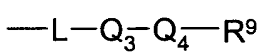

(ix)

und

**$R^4$** ein Rest ausgewählt aus der Gruppe bestehend aus Wasserstoff, $-OR^6$, $-C(=O)R^6$, $-C(=O)OR^6$, $-C(=O)NR^6R^7$, $-NR^6R^7$, $-NR^6C(=O)R^7$, $-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen, oder ein Rest ausgewählt aus der Gruppe bestehend aus gegebenenfalls einfach oder mehrfach substituiertem $C_{1-8}$-Alkyl, $C_{2-10}$-Akenyl, $C_{2-10}$-Alkinyl, $C_{3-8}$-Cycloalkyl, Aryl, Heterocyclyl und Heteroaryl, wobei der/die Substituent(en) gleich oder verschieden sein können und ausgewählt werden aus der Gruppe bestehend aus $C_{1-8}$-Alkyl, Halogen, $-NO_2$, $-OR^6$, $-C(=O)R^6$, $-C(=O)OR^6$, $-C(=O)NR^6R^7$, $-NR^6R^7$, $-NR^6C(=O)R^7$,

$-NR^6C(=O)OR^7$, $-NR^6C(=O)NR^7R^8$, $-NR^6SO_2R^7$, $-N=CR^6R^7$, $-SR^6$, $-SOR^6$, $-SO_2R^6$, $-SO_2NR^6R^7$, $-NR^6SO_2NR^7R^8$, $-OSO_2NR^7R^8$ und Pseudohalogen; und

**$R^5$** Wasserstoff, Halogen, $-CF_3$, $C_{1-3}$-Alkyl oder $-OR^6$; und

**$R^6$, $R^7$** und **$R^8$** jeweils unabhängig voneinander Wasserstoff oder ein Rest ausgewählt aus der Gruppe bestehend aus gegebenenfalls einfach oder mehrfach substituiertem $C_{1-5}$-Alkyl, $C_{2-5}$-Alkenyl, $C_{2-5}$-Alkinyl, $C_{3-10}$-Cycloalkyl, Aryl, Heterocyclyl und Heteroaryl, wobei der/die Substituenten gleich oder verschieden sein können und ausgewählt werden aus der Gruppe bestehend aus $C_{3-10}$-Cycloalkyl, Aryl, Heterocyclyl, Heteroaryl, Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}C(=O)R^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**L** eine Bindung oder ein Rest ausgewählt aus der Gruppe bestehend aus gegebenenfalls einfach oder mehrfach substituiertem $C_{1-16}$-Alkyl, $C_{2-16}$-Alkenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$Q_3$** und **$Q_4$** unabhängig voneinander eine Bindung oder ein Rest ausgewählt aus der Gruppe bestehend aus gegebenenfalls einfach oder mehrfach substitutierten monooder bizyklische Heterocyclyl wobei der/die Substituent(en) gleich oder verschieden sein können und ausgewählt werden aus der Gruppe bestehend aus Methyl, Ethyl, Halogen, $-NH_2$, -OH und Pseudohalogen; und

**$R^9$** ein Rest ausgewählt aus der Gruppe bestehend aus gegebenenfalls einfach oder mehrfach substituiertem $C_{1-16}$-Alkyl, $C_{2-16}$-Akenyl, $C_{2-16}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen-, $-NO_2$, $-OR^{10}$, $-C(=O)R^{10}$, $-C(=O)OR^{10}$, $-C(=O)NR^{10}R^{11}$, $-NR^{10}R^{11}$, $-NR^{10}COR^{11}$, $-NR^{10}C(=O)OR^{11}$, $-NR^{10}C(=O)NR^{11}R^{12}$, $-NR^{10}C(=O)ONR^{11}R^{12}$, $-NR^{10}SO_2R^{11}$, $-N=CR^{10}R^{11}$, $-SR^{10}$, $-SOR^{10}$, $-SO_2R^{10}$, $-SO_2NR^{10}R^{11}$, $-NR^{10}SO_2NR^{11}R^{12}$, $-OSO_2NR^{10}R^{11}$ und Pseudohalogen; und

**$R^{10}$, $R^{11}$** und **$R^{12}$** jeweils unabhängig voneinander Wasserstoff oder ein Rest ausgewählt aus der Gruppe bestehend aus gegebenenfalls einfach oder mehrfach substituiertem $C_{1-8}$-Alkyl, $C_{2-8}$-Alkenyl, $C_{2-8}$-Alkinyl, $C_{3-10}$-Cycloalkyl, Aryl, Heterocyclyl und Heteroaryl, wobei der/die Substituent(en) gleich oder verschieden sein können und ausgewählt werden aus der Gruppe bestehend aus Halogen, $-NH_2$, -OH und Pseudohalogen;

gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und
mindestens eine weitere Wirksubstanz ausgewählt aus der Gruppe bestehend aus zytostatischen Wirksubstanzen, zytotoxische Wirksubstanzen, Steroide und Antikörper, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Europäisches Patentamt

# EUROPÄISCHER RECHERCHENBERICHT

Nummer der Anmeldung
EP 04 01 1911

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| E | WO 2004/080980 A1 (NOVARTIS A.-G., SWITZ.; NOVARTIS PHARMA G.M.B.H.) 23. September 2004 (2004-09-23) siehe e.g. Beispiele 7-1, 7-3, 7-14, 7-25, 9-2, 9-3, 9-4, 10-2, 11-2, 15-1; ----- | 1-7, 9-11, 13-15 | C07D239/48 A61K31/505 A61P29/00 A61P31/12 A61P35/00 A61P37/00 |
| Y | WO 00/12485 A (BREAULT GLORIA ANNE ; PEASE JANET ELIZABETH (GB); ZENECA LTD (GB)) 9. März 2000 (2000-03-09) siehe Ansprüche 1 und 10 bis 13 und e.g. Beispiel 97 ----- | 1-15 | |
| Y | US 6 593 326 B1 (BRADBURY ROBERT H ET AL) 15. Juli 2003 (2003-07-15) siehe Ansprüche 1, 5, 8, 12 und 13 und Beispiele 195 bis 215 ----- | 1-15 | |
| Y | WO 00/27825 A (AKEN KOEN JEANNE ALFONS VAN ; KOYMANS LUCIEN MARIA HENRICUS (BE); HEER) 18. Mai 2000 (2000-05-18) siehe Ansprüche 1, 5, 7 und 8 und die Definition von R2a als Mono- oder Di(methylamino)carbonyl; ----- | 1-9,13, 15 | RECHERCHIERTE SACHGEBIETE (Int.Cl.7) C07D |

Der vorliegende Recherchenbericht wurde für alle Patentansprüche erstellt

| Recherchenort | Abschlußdatum der Recherche | Prüfer |
|---|---|---|
| München | 5. November 2004 | Traegler-Goeldel, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

2

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 04 01 1911

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-2004

| Im Recherchenbericht angeführtes Patentdokument | Datum der Veröffentlichung | Mitglied(er) der Patentfamilie | Datum der Veröffentlichung |
|---|---|---|---|
| WO 2004080980 A1 | 23-09-2004 | KEINE | |
| WO 0012485 A | 09-03-2000 | AU 5438299 A | 21-03-2000 |
| | | EP 1107957 A1 | 20-06-2001 |
| | | WO 0012485 A1 | 09-03-2000 |
| | | JP 2002523497 T | 30-07-2002 |
| US 6593326 B1 | 15-07-2003 | AT 277020 T | 15-10-2004 |
| | | AU 763091 B2 | 10-07-2003 |
| | | AU 1874300 A | 31-07-2000 |
| | | BR 9916590 A | 23-10-2001 |
| | | CA 2352896 A1 | 06-07-2000 |
| | | CN 1335838 T | 13-02-2002 |
| | | DE 69920509 D1 | 28-10-2004 |
| | | EP 1140860 A1 | 10-10-2001 |
| | | WO 0039101 A1 | 06-07-2000 |
| | | JP 2002533446 T | 08-10-2002 |
| | | NO 20013038 A | 22-08-2001 |
| | | NZ 512118 A | 29-08-2003 |
| | | ZA 200104413 A | 29-08-2002 |
| WO 0027825 A | 18-05-2000 | AT 233740 T | 15-03-2003 |
| | | AU 762523 B2 | 26-06-2003 |
| | | AU 6200899 A | 29-05-2000 |
| | | BG 105418 A | 30-11-2001 |
| | | BR 9915552 A | 14-08-2001 |
| | | CA 2350801 A1 | 18-05-2000 |
| | | CN 1322198 T | 14-11-2001 |
| | | CZ 20011533 A3 | 17-10-2001 |
| | | DE 69905683 D1 | 10-04-2003 |
| | | DE 69905683 T2 | 18-03-2004 |
| | | DK 1002795 T3 | 30-06-2003 |
| | | EA 4049 B1 | 25-12-2003 |
| | | EE 200100252 A | 15-10-2002 |
| | | WO 0027825 A1 | 18-05-2000 |
| | | EP 1270560 A1 | 02-01-2003 |
| | | EP 1002795 A1 | 24-05-2000 |
| | | ES 2193664 T3 | 01-11-2003 |
| | | HK 1025330 A1 | 13-06-2003 |
| | | HR 20010161 A1 | 28-02-2002 |
| | | HU 0104177 A2 | 28-03-2002 |
| | | ID 28376 A | 17-05-2001 |
| | | JP 2002529456 T | 10-09-2002 |
| | | NO 20011696 A | 04-04-2001 |
| | | NZ 511116 A | 29-08-2003 |
| | | PL 347586 A1 | 08-04-2002 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 04 01 1911

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-2004

| Im Recherchenbericht angeführtes Patentdokument | Datum der Veröffentlichung | Mitglied(er) der Patentfamilie | Datum der Veröffentlichung |
|---|---|---|---|
| WO 0027825 A | | PT 1002795 T | 31-07-2003 |
| | | SI 1002795 T1 | 31-10-2003 |
| | | SK 6032001 A3 | 07-01-2002 |
| | | TR 200101306 T2 | 22-10-2001 |
| | | US 2003114472 A1 | 19-06-2003 |
| | | US 2004039005 A1 | 26-02-2004 |
| | | ZA 200103769 A | 12-08-2002 |

EPO FORM P0461

Für nähere Einzelheiten zu diesem Anhang : siehe Amtsblatt des Europäischen Patentamts, Nr.12/82